# EUROPEAN PATENT APPLICATION

(11) **EP 4 704 102 A1**
(43) Date of publication of application: **04.03.2026**
(21) Application number: 24197622.4
(22) Date of filing: 30.08.2024
(51) Int. Cl.: G16H 30/20, G16H 30/40

(54) **METHODS AND SYSTEMS FOR PROVIDING AN IMAGE ACQUISITION INFORMATION OF A MEDICAL IMAGE**

(71) Applicant: Siemens Healthineers AG, 91301 Forchheim (DE)
(72) Inventor: Allen-Raffl, Simon, West Chester, 19382 (US); Gibson, Eli, Plainsboro, 08536 (US); Hermosillo Valadez, Gerardo, West Chester, 19382 (US); Marchi, Cristina, 90542 Forth, Eckental (DE); Shinagawa, Yoshihisa, Downingtown, 19335 (US); Yerebakan, Halid, Carmel, 46033 (US)
(74) Representative: Siemens Healthineers Patent Attorneys

(57) **Abstract**

Computer-implemented methods and systems for providing an image acquisition information of a medical image are provided. The methods and systems implement a plurality of steps. One step is directed to obtaining the medical image. Another step is directed to extracting image data from the medical image. Another step is directed to obtaining non-image data associated with the image data of the medical image. Another step is directed to providing a first trained function configured to determine an image acquisition information based on non-image data of medical images. Another step is directed to providing a second trained function configured to determine an image acquisition information based on image data associated with image data of the medical image. Another step is directed to determining a first image acquisition information by applying the first trained function to the non-image data. Another step is directed to determining a second image acquisition information by applying the second trained function to the image data. Another step is directed to determining the image acquisition information based on the first image acquisition information and the second image acquisition information. Another step is directed to providing the image acquisition information.

## Description

Independent of the grammatical term usage, individuals with male, female or other gender identities are included within the term.

Aspects of the invention relate to methods and systems for providing an image acquisition information of medical images. In particular, aspects of the invention relate to systems and methods for deriving an image acquisition information from image data and non-image data of medical images. Further, aspects of the invention relate to the usage of the image acquisition information for processing the medical image, in particular, for deriving a medical diagnosis.

Automation of workflows is still a challenge in the processing of medical images for arriving at a possible diagnosis of the patient. Thereby, image processing starts with tasks which might appear trivial at first sight such as the selection of appropriate displaying settings or the placement of the image data on a displaying screen of the reviewing physician. It goes on with more subtle decisions such as the selection of the correct image processing, in particular, computer aided detection (CAD) tools, the selection of auxiliary data, the routing of data to the competent physician, the selection of appropriate reporting templates for creating the final medical report, and so forth.

Automating these and other steps are difficult as these steps crucially depend on the underlying medical image. To put it differently, different images may require a very different treatment in a reading and reporting workflow. The characteristics of the medical image may be summed up in the image acquisition information indicating, e.g., the imaging modality and the settings used, or the body part displayed.

One option could be to read this information from the documentation available for the medical images. One issue with this approach is that there often is too little information documented for the image acquisition parameters which would allow to make an educated decision regarding automated processing steps. Further, regarding the image studies to be automatically processed there often is huge variety. This can come from different available acquisition and/ or reconstructions technologies, different modalities, or even different preferences in the acquisition.

What is more, in the clinical reality, the documentation of imaging parameters and imaged organs is not only scarce but oftentimes also not existent or flat wrong. Due to the time pressure in the clinical routine, there often are dummy entries in the protocols which cannot be used.

As a consequence, any straight-forward automation which is (only) based on what is expressively documented in a patient case often leads to inappropriate results. This not only consumes system resources but may lead to extra work at the side of the users - which is completely contrary to the original intention of automizing the workflow in radiology reading and reporting.

To address suchlike problems, DE 10 2024 201 496 proposes to automatically extract relevant image acquisition information by transforming the medical image data into the frequency domain to obtain a k-space image and to apply a trained function to the k-space image data.

While such methods generally perform well for specific tasks, the inventors have found that the accuracy for correctly predicting subtle differences for more general use cases is not sufficient. Further, suchlike methods systematically do not utilize all of the information available as they are only designed to process image data.

This is even more important as the type of the image data and non-image data can vary as well as the image acquisition parameters. Such image acquisition parameters as the CT imaging protocol or the MR sequence are decisive for many tasks which can be potentially automated. This concerns potential post-processing steps (e.g., convolution kernels), image enhancement steps (applying the correct window, e.g., bone), tool selection (e.g., lung CAD for lung-related image data), the hanging order, or prior image selection. The latter is of particular importance in cases where a long-term observation of patients is required to determine the development of certain diseases like the growth of tumors.

Accordingly, it is an object of embodiments of the invention to improve the processing of medical images for obtaining a medical diagnosis. In particular, it is an object of embodiments of the invention to improve the provision of image acquisition information of a medical image, in particular, for a subsequent usage in the processing of the medical image aiming at obtaining a medical diagnosis based on the medical image.

This object is solved by a computer-implemented method for providing an image acquisition information of a medical image, a system for providing an image acquisition information of a medical image corresponding computer-program products, and computer-readable storage media according to the main claims. Alternative and/or preferred embodiments are object of the dependent claims.

In the following, the technical solution according to the present invention is described with respect to the claimed apparatuses as well as with respect to the claimed methods. Features, advantages, or alternative embodiments described herein can likewise be assigned to other claimed objects and vice versa. In other words, claims addressing the inventive method can be improved by features described or claimed with respect to the apparatuses. In this case, e.g., functional features of the method are embodied by objective units or elements of the apparatus.

The technical solution will be described both with regard to methods and systems for providing an updated machine learned function and also with regard to methods and systems for providing training or test data for updating a machine learned system. Features and alternate forms of embodiments of data structures and/or functions for methods and systems for providing machine learned functions can be transferred to analogous data structures and/or functions for methods and systems for providing training or test data. Analogous data structures can, in particular, be identified by using the prefix "training". Furthermore, the prediction functions used in methods and system for providing information can, in particular, have been adjusted and/or trained and/or provided by methods and systems for adjustment of prediction functions.

According to an aspect, a computer-implemented method for providing an image acquisition information of a medical image is provided. The method comprises a plurality of steps. One step is directed to obtaining the medical image. Another step is directed to extracting image data from the medical image. Another step is directed to obtaining non-image data associated with the image data of the medical image. Another step is directed to providing a first trained function configured to determine an image acquisition information based on non-image data of medical images. Another step is directed to providing a second trained function (different from the first trained function) configured to determine an image acquisition information based on image data associated with image data of the medical image. Another step is directed to determining a first image acquisition information by applying the first trained function to the non-image data. Another step is directed to determining a second image acquisition information by applying the second trained function to the image data. Another step is directed to determining the image acquisition information based on the first image acquisition information and the second image acquisition information. Another step is directed to providing the image acquisition information.

The medical image may be a two-dimensional image. Further, the medical image data set may be a three-dimensional image. Further, the medical image may be a four-dimensional image, where there are three spatial and one time-like dimensions. Further, the medical image data set may comprise a plurality of individual medical images.

The medical image comprises image data, for example, in the form of a two- or three-dimensional array of pixels or voxels. Such arrays of pixels or voxels may be representative of color, intensity, absorption or other parameters as a function of two or three-dimensional position, and may, for example, be obtained by suitable processing of measurement signals obtained by a medical imaging modality or image scanning facility.

The medical image may depict a body part of a patient. Accordingly, it may contain two or three-dimensional image data of the patient's body part. The medical image may be representative of an image volume or a cross-section through the image volume. The patient's body part may be comprised in the image volume.

A medical imaging modality corresponds to a system used to generate or produce medical image data. For example, a medical imaging modality may be a computed tomography system (CT system), a magnetic resonance system (MR system), an angiography (or C-arm X-ray) system, a positron-emission tomography system (PET system) or the like. Specifically, computed tomography is a widely used imaging method and makes use of "hard" X-rays produced and detected by a spatially rotating instrument. The resulting attenuation data (also referred to as raw data) is processed by a computed analytic software producing detailed images of the internal structure of the patient's body parts. The produced sets of images are called CT-scans which may constitute multiple series of sequential images to present the internal anatomical structures in cross sections perpendicular to the axis of the human body. Magnetic Resonance Imaging (MRI), to provide another example, is an advanced medical imaging technique which makes use of the effect magnetic field impacts on movements of protons. In MRI machines, the detectors are antennas, and the signals are analyzed by a computer creating detailed images of the internal structures in any section of the human body.

A medical image may comprise a plurality of image slices. The slices respectively show a cross-sectional view of the image volume. The slices may comprise a two-dimensional array of pixels or voxels as image data. The arrangement of slices in the medical image data set may be determined by the imaging modality or by any post-processing scheme used. Further, slices may artificially be defined in the imaging volume spanned by the medical image data set.

Optionally, this may happen as a function of the image data comprised in the medical image data set in order to optimally pre-process the medical image data set for the ensuing diagnostic workflow.

Further, a medical image may comprise a plurality of image series each representing an individual scan of the patient. Each series may relate to a 2D or 3D imaged area.

The medical image may be stored in a standard image format such as the Digital Imaging and Communications in Medicine (DICOM) format and in a memory or computer storage system such as a Picture Archiving and Communication System (PACS), a Radiology Information System (RIS), and the like. Whenever DICOM is mentioned herein, it shall be understood that this refers to the "Digital Imaging and Communications in Medicine" (DICOM) standard, for example according to the DICOM PS3.1 2020c standard (or any later or earlier version of said standard).

The non-image data may comprise non-image data included in the medical image and non-image data separate from the medical image. The non-image information may comprise structured and/or unstructured natural language text. The non-image information may comprise one or more data elements, in particular, text documents. The non-image information may comprise supplementary information to the image data of the medical image.

A medical image data file, a DICOM file for example, usually contains a header wherein information, in particular, non-image data is stored in a text form. The medical image may comprise non-image data, for example in the form of a one- or multi-dimensional array of characters. Such arrays of characters may, for example, be obtained by logging of imaging processes and/or suitable processing of measurement signals obtained by a medical imaging modality or image scanning facility. Another example of non-image data, in particular contained in the header of the medical image, may be image annotation data, which may include labels, tags, captions, or comments added to the image by medical professionals, researchers, or patients. The image annotation data may provide semantic information about the image content, such as the identification of anatomical structures, pathological findings, or measurement values.

The non-image data associated with the image data of the medical image may, however, be also contained in another file, for example an electronic medical record of the patient (EMR).

According to some examples, the non-image data may comprise one or more of the following data elements:
- a prior medical report of the patient, and/or
- a-priori knowledge of at least one type of medical problem the patient is suspected to have, and/or
- an indication of a diagnostic task to be performed based on the medical image data set for the patient, and/or
- an electronic health record of the patient.

The medical image may be obtained from a Picture Archiving and Communication System (PACS). Thereby, the PACS may store the medical images in a post-processed version after acquisition. The post-processed version may relate to readily viewable version in an image viewer and not to raw data.

In general, a trained function, in particular, of the like of the first or second trained function, mimics cognitive functions that humans associate with other human minds. In particular, by training based on training data, the machine-learned function is able to adapt to new circumstances and to detect and extrapolate patterns. Other terms for machine-learned function, may be machine-learned function, trained machine learning model, trained mapping specification, mapping specification with trained parameters, function with trained parameters, algorithm based on artificial intelligence, or machine learned algorithm.

In general, parameters of a machine-learned function can be adapted by means of training. In particular, supervised training, semi-supervised training, unsupervised training, reinforcement learning and/or active learning can be used. Furthermore, representation learning (an alternative term is "feature learning") can be used. In particular, the parameters of the machine-learned function can be adapted iteratively by several steps of training.

In particular, a trained function can comprise a neural network, a support vector machine, a decision tree and/or a Bayesian network, and/or the trained function can be based on k-means clustering, Q-learning, genetic algorithms and/or association rules. In particular, a neural network can be a deep neural network, a convolutional neural network or a convolutional deep neural network. Furthermore, a neural network can be an adversarial network, a deep adversarial network and/or a generative adversarial network.

The trained function may be generally configured to determine image acquisition information based on image data and/or non-image data obtained from the medical image. For instance, the trained function may be configured to extract one or more features from an image and a text and map/classify these features into a feature space associated with different image acquisition parameters for determining which acquisition information the image data and/or non-image data indicates. Thus, the trained function may comprise a feature extractor and a classifier. In particular, the feature extractor and the classifier may be implemented as a neural network, in particular, a convolutional neural network, with some network layers trained to extract features and other network layers being trained to provide a classification according to the most likely image acquisition information. Further, the image acquisition information may be obtained by applying a natural language processing function to the non-image information in particular with the task to identify image acquisition information in the non-image information. According to some examples, the natural language processing function may be based on a large language model.

The first trained function may generally be configured to process natural language text and predict image acquisition information on that basis. One example of a trained function, in particular a first trained function, for the non-image data analysis, in particular the determination of the first image acquisition information, is a language model, such as a transformer-based model, which can process natural language texts and extract relevant information from them. For example, a language model, such as BERT or the language models of the GPT family, can be provided as a first trained function to determine an image acquisition information based on non-image data. The first trained function, in particular a language model, can be trained on a large corpus of medical texts, such as reports, annotations, or labels, that contain information about the imaging modality, the body part, and the findings of the medical images. The first trained function, in particular the language model, may encode the non-image data into a vector representation and output a first image acquisition information. Further, the first trained function, in particular the language model, may output an information or score, about the accuracy of the non-image data, in particular a first confidence score.

The second trained function may generally be configured to process image data and predict image acquisition information on that basis. One example of a trained function, in particular a second trained function, for analyzing the image data, in particular the determination of the second image acquisition information, is a ResNet convolutional neural network, which can process pixel values and extract features from them. The second trained function, in particular the convolutional neural network, can be trained on a large dataset of medical images, such as X-rays, CT scans, or MRIs, which are labeled with information about the imaging modality, the body part, and the findings of the images. The second trained function, in particular the convolutional neural network, can then generate a second image acquisition information based on the features extracted from the image data.

If the non-image data is sufficient, complete and/or accurate, which may be determined by the user and/or a further or the first trained function, the step of determining the image acquisition information may solely be based on the first image acquisition information. This means that the image data does not need to be processed further, and the image acquisition information can be determined based on the first image acquisition information. However, if the non-image data is insufficient, incomplete and/or inaccurate, the image data needs to be processed by the second trained function.

According to some examples first and second trained functions are different from one another. Further, according to some examples, first and second trained functions may be independent from one another. According to some examples, independent may mean that first and second trained functions may respectively have a different architecture (e.g., a transformer architecture in the first trained function as compared to a FocalNet in the second trained function) and/or have been trained independently from one another using different training data. This may have the advantage of independent results allowing for a cross-validation.

As an alternative, first and second trained functions may also be integrated in one trained function, e.g., as different branches of a network. This may allow for a more efficient training as shared weights between the branches may allow for a faster convergence towards the local optimum.

The image acquisition information (and, therewith the first and second image acquisition information) may relate to an image acquisition procedure with a medical imaging modality with which image acquisition procedure the medical image has been acquired. The image acquisition information as well as the first and second image acquisition information may comprise a defined data structure, in particular comprising a plurality of, in particular defined data items. According to some examples, each data item may define an image acquisition parameter, such as the modality used, the modality settings used, the body part examined, the image acquisition protocol, the diagnostic background and the like. According to some examples, the image acquisition information, in particular the data items of the image acquisition information, may comprises a modality, an anatomy, and/or a procedure based on which the medical image has been acquired. According to some examples, the image acquisition information comprises one or more image acquisition parameters with which or based on which the medical image has been acquired, such as a type of the medical imaging modality used, settings of the medical imaging modality, or a type of the medical image data.

The image acquisition information which is generated based on first and second image acquisition information may be regarded as a consolidated image acquisition information which has been generated based on a combination of first and second image acquisition information. The image acquisition information and the first and second image acquisition information may have the same structure, enabling a reasonable comparison of first and second image acquisition information to form the (consolidated and final) image acquisition information.

According to some examples, determining the image acquisition information comprises comparing the first image acquisition information and the second image acquisition information. Further, determining the image acquisition information may comprise comparing individual data items of first and second image acquisition information (so as to determine if individual data item sufficiently correspond between the first and second image acquisition information). Further, determining the image acquisition information may comprise integrating data items which have been determined to sufficiently correspond in the image acquisition information. According to some examples, the data items may comprise any one of a modality used, a scan parameter, a reason for the exam, an anatomy and so forth. Further, determining the image acquisition information may comprise assigning a score, in particular a confidence score, to each of the first and second image acquisition information based on the quality, completeness, and/or accuracy of the data sources (if individual data item are divergent between the first and second image acquisition information). According to some examples, one image acquisition information of the first and second image acquisition information with the highest score is selected as the image acquisition information for the medical image.

Alternatively, the method may comprise a (third) trained function, which may compare the first and second image acquisition information and determine their consistency or discrepancy. the third trained function may perform a fusion of the first and second image acquisition information and generate an image acquisition information that incorporates the most relevant and consistent information from both data sources. This may involve resolving any conflicts or ambiguities between the first and second image acquisition information and weighting the information according to their importance and reliability.

The inventors have recognized that by applying one or more trained function to image data and the non-image data of the medical image data the image acquisition information becomes better accessible. With that, the image acquisition information may be obtained in a more secure manner especially in cases where information is not documented either in the available medical record of the patient and/or the meta-data of the medical image or not derivable form the image data. In turn, the application of a trained function on two information sources allows for a more efficient and robust automation of the process and/or workflow directed to the provision of a medical diagnosis based on the medical image done by a user.

In principle, all suited trained functions may be used for obtaining the image acquisition information from the image data and/or non-image data.

According to some examples, however, the first trained function comprises at least one of: a transformer network, and/or LLM.

According to some examples, however, the second trained function comprises at least one of: a convolutional neural network, a transformer network, in particular, a vision transformer, and/or a Focal Net.

A convolutional neural network is a neural network that uses a convolution operation instead general matrix multiplication in at least one of its layers (so-called "convolutional layer"). In particular, a convolutional layer performs a dot product of one or more convolution kernels with the convolutional layer's input data / im-age, wherein the entries of the one or more convolution kernel are the parameters or weights that are adapted by training. In particular, one can use the Frobenius inner product and the ReLU activation function. A convolutional neural network can comprise additional layers, e.g., pooling layers, fully connected layers, and normalization layers.

For an example for the general usability of convolutional neural networks for deriving image acquisition parameters, reference is made to van der Voort et al., DeepDicomSort: An Automatic Sorting Algorithm for Brain Magnetic Resonance Imaging Data, in Neuroinformatics, 2021, Jan, 19(1):159-184, doi: 10.1007/s12021-020-09475-7, the contents of which are herein included by reference in their entirety. While being applied on real image data in van der Voort et al., the cited application DE 10 2024 201 496 offers an architecture that may also be applied to k-space images. The contents of DE 10 2024 201 496 are herein included by reference in their entirety as well.

By using convolutional neural networks, input image data and non-image data can be processed in a very efficient way, because a convolution operation based on different kernels can extract various features from both text and image data. By adapting the weights of the convolution kernel, the relevant features can be readily found during training. Furthermore, based on the weight-sharing in the convolutional kernels, less parameters need to be trained, which prevents overfitting in the training phase and allows to have faster training or more layers in the network, improving the performance of the network. The same feature detection logic can be applied across different types of data, which can lead to more efficient learning and generalization. This is because the network can learn to recognize patterns that are common to both non-image and image data, which can be particularly useful in applications where both visual and textual information are important.

A transformer network is a neural network architecture generally comprising an encoder, a decoder, or both an encoder and decoder.

In some instances, the encoders and/or decoders are composed of several corresponding encoding layers and decoding layers, respectively. Within each encoding and decoding layer is an attention mechanism. The attention mechanism, sometimes called self-attention, relates data elements (such as words or pixels) within a series of data elements to other data elements within the series.

The encoder, in particular, may be configured to transform the input image data into a numerical representation. The numerical representation may comprise a vector per input token (e.g., per image and/or text patch). The encoder may be configured to implement an attention mechanism so that each patch is affected by the other patches in the input. In particular, the encoder may be configured such that the representations resolve the desired output, i.e., the image acquisition information of the trained function.

The decoder, in particular, may be configured to transform an input into a sequence of output tokens. In particular, the decoder may be configured to implement a masked self-attention mechanism so that each vector of a token is affected only by the other tokens to one side of a sequence. Further, the decoder may be auto-regressive meaning in that intermediate results (such as a previously predicted sequence of tokens) are fed back.

According to some examples, the output of the encoder is input into the decoder.

Further, the transformer network may comprise a classification module or unit configured to map the output of the encoder or decoder to a set of learned outputs in the form of the image acquisition information.

In particular, the transformer network may be embodied as a vision transformer. The vision transformer may be configured to break down input image data or non-image data into patches and tokenize them (extracting representation vectors), before applying the tokens to a standard transformer architecture. The vision transformer may comprise an attention mechanism configured to repeatedly transform representation vectors of image and/or text patches for incorporating more and more semantic relations between image and/or text patches in an image.

According to some examples, the vision transformer may be obtained by training a masked autoencoder. A masked auto-encoder comprises two vision transformers put end-to-end. The first one takes in image patches with positional encoding, and outputs vectors representing each patch. The second one takes in vectors with positional encodings and outputs image patches again. During training, both vision transformers are used. An image is cut into patches. The second vision transformer takes the encoded vectors and outputs a reconstruction of the full image. During use, the first vision transformer may be used as encoder and/or the second vision transformer may be used as generative AI function.

For a review on transformer networks, reference is made to Vaswani et al., "Attention Is All You Need", in arXiv: 1706.03762, June 12, 2017, the contents of which are herein included by reference in their entirety.

An advantage of transformer networks is that, due to the attention mechanism, transformer networks can efficiently deal with long-range dependencies in input data. Further, encoders used in transformer networks are capable of processing data in parallel which saves computing resources in inference. Moreover, decoders of transformer networks, due the auto-regression, are able to iteratively generate a sequence of output tokens with great confidence.

FocalNet is short for focal modulation network. A FocalNet is a neural network that uses a focusing mechanism to enable the model's interaction with the input, e.g. an image. Specifically, FocalNets use a lightweight element-wise multiplication as a focusing operator to see or interact with the input with the proposed modulator. The modulator is computed with a focal aggregation procedure in two steps: focal contextualization to extract contexts from local to global regions at different granularity levels and gated aggregation to condense all context features at different granularity levels into the modulator. For a review on FocalNets, reference is made to Yang et al., Focal Modulation Networks, arXiv:2203.11926, the contents of which are herein included by reference in their entirety.

The inventors have recognized that FocalNet may outperform attention-based neural networks when dealing with image data. FocalNet can also handle both non-image data and image data seamlessly with its modulator, which can focus on different regions and levels of granularity in the input. This may enable FocalNet to extract more relevant and robust features from the image data and non-image data, which may be noisy or incomplete.

LLM stands for large language model, a type of computational model that can perform various natural language processing tasks, such as text generation and classification. LLMs are based on language models, which can learn statistical patterns of natural languages from massive amounts of text data. LLMs can achieve general-purpose language generation by using a self-supervised or semi-supervised training process, in which they optimize their parameters to predict the next token or word given an input text. LLMs can be used as (generative) trained functions to process and/or produce diverse texts for different purposes. LLMs usually comprise artificial neural networks that use the transformer architecture. The transformer architecture allows LLMs to efficiently process and generate large-scale text data by using an attention mechanism that captures the long-range dependencies and semantic relations between tokens or words.

According to some examples, the LLM may be based on an encoder-only transformer network structure. The encoder-only transformer network structure comprises a stack of encoder layers that receive the input text as a sequence of tokens and output a sequence of representation vectors. The encoder layers use self-attention mechanisms to attend to the input tokens and encode their semantic and syntactic information. The encoder-only transformer network structure does not include a decoder layer that generates output tokens from the representation vectors. Instead, the representation vectors are used as inputs for different downstream tasks, such as classification or regression. The encoder-only transformer network structure may have advantages in terms of efficiency and scalability, as it can process large amounts of text data without generating new text data. The encoder-only transformer network structure may also have advantages in terms of accuracy and robustness, as it can learn general-purpose language representations that can be applied to medical domains and tasks.

According to some examples, the medical image is a 3D medical image, and the method further comprises obtaining an image slice of the medical image, wherein the image data is extracted from the obtained image slice.

Thereby, obtaining the image slice may comprise selecting the image slice from a plurality of slices comprised in the medical image or defining the image slice in the 3D medical image.

According to some examples, obtaining the image slice may comprise determining a suitability measure (or quality score) of a plurality of candidate slices of the medical image, wherein the suitability measure indicates a suitability of the slice for the steps of transforming and the ensuing determining of the image acquisition information. According to some examples the suitability measure may be based on a position of the slice in the medical image (wherein marginal slices may have a lower suitability than more central slices) and/or an image content of the slice (wherein image slices with imaging artifacts may have a lower suitability).

According to some examples, the suitability measure may be determined by a further trained function which has been configured to obtain the image slice. The further trained function may be based on a convolutional neural network. Further, the further trained function may be part of the trained function. The further trained function may be trained by processing a plurality of slices from the medical image with the trained function and comparing the result to a ground truth for the image acquisition information. The result of the comparison may be fed back the further trained function for optimizing the slice obtaining process.

By obtaining the image slice, information can be pre-selected from the medical image which represents the medical image, and which is suited for the ensuing processing. This may increase the quality of the processing results and the efficiency of the method.

According to some examples, data, in particular non-image data, associated with the image data of the medical image comprises various data elements and obtaining the non-image data associated with the image data of the medical image further comprises obtaining a data element of the data, wherein the non-image data is obtained from the obtained data element.

Thereby, obtaining the data element may comprise selecting the data element from a plurality of data elements comprised in the non-image data. In particular, the non-image data may comprise text documents and the step of obtaining a of the non-image data may comprise selecting a text segment or defining a text segment in the data element, in particular the text document.

According to some examples, obtaining the data element may comprise determining a relevance measure (or similarity score) of a plurality of candidate data elements of the non-image data, wherein the relevance measure indicates a relevance of the segment for the steps of transforming and the ensuing determining of the image acquisition information. According to some examples, the relevance measure may be based on a type of the data element in the non-image data (wherein for example a data element including a prior medical report of the patient may have a higher relevance than a data element including a-priori knowledge of at least one type of medical problem the patient is suspected to have) and/or a text content of the data element (wherein data elements or segments of data elements with keywords or phrases related to the image acquisition information may have a higher relevance).

According to some examples, the relevance measure may be determined by a further trained function which has been configured to obtain the data element. The further trained function may be based on a natural language processing model. Further, the further trained function may be part of the trained function. The further trained function may be trained by processing a plurality of data elements from non-image data, for example segments from text documents associated with the medical image, with the trained function and comparing the result to a ground truth for the image acquisition information. The result of the comparison may be fed back to the further trained function for optimizing the data element obtaining process.

By obtaining the data element, information can be pre-selected from the non-image data which is suited for the ensuing processing. This may enhance the quality of the processing results and the efficiency of the method. Moreover, the non-image data can comprise various and numerous data elements and information, some of which might be significant, some of which might be less valuable regarding their relevance and accuracy. Obtaining relevant and accurate data element from the non-image data further contributes to a reliable determination of the image acquisition information.

According to an aspect, the image acquisition information comprises an image acquisition parameter and/or an information about a body part depicted in the medical image.

An image acquisition parameter may comprise the type and/or make of the imaging modality used and/or control parameter settings of the imaging modality used during the acquisition of the medical image. To provide an example, image acquisition parameters may comprise the following information: type Chest-CT scan, bolus agent: xyz, modality: Siemens Healthineers CT scanner, model number: 12345, kilovoltage peak: xxx, milliampere seconds: yyy. The information about the body part may comprise an indication of the body part depicted and/or the body compartments therein comprised, and/or an indication of findings comprised in the body part/compartments. To provide an example, information about the body part may comprise: chest area, showing the lung, rib cage, spine of the patient with a lung nodule in the upper left lung lobe. To provide another example, the image acquisition information can specify what kind of image weighting and which magnetic resonance sequence has been used for generating the medical image. In particular, the image acquisition information can provide insights in the spin-echo sequence used, e.g., whether a BLADE-like or HASTE-like sequence was used.

With the image acquisition parameters and/or information about body parts valuable information is provided which determines the further processing steps in the further diagnostic image processing workflow. With that, these steps can be more readily triggered automatically without user input.

According to an aspect, the medical image has been acquired with a magnetic resonance acquisition procedure using a magnetic resonance medical imaging modality, and the image acquisition parameter relates to the image weighting and/or the magnetic resonance sequence used in the acquisition procedure. In other words, determining the image acquisition parameter comprises classifying the medical image according to the image weighting and/or the magnetic resonance sequence used.

The image weighting may indicate which relaxation effect the magnetic resonance imaging was focused on. Each tissue returns to its equilibrium state after excitation by independent relaxation processes of T1 (spin-lattice; that is, magnetization in the same direction as the static magnetic field) and T2 (spin-spin; transverse to the static magnetic field). To create a T1-weighted image, magnetization is allowed to recover before measuring the MR signal by changing the repetition time. This image weighting is useful for assessing the cerebral cortex, identifying fatty tissue, characterizing focal liver lesions, and in general, obtaining morphological information, as well as for post-contrast imaging. To create a T2-weighted image, magnetization is allowed to decay before measuring the MR signal by changing the echo time. This image weighting is useful for detecting edema and inflammation, revealing lesions and abnormalities.

Within the T1/T2 there may be more subtle variations. The T2* weighting builds on a distribution of resonance frequencies around the ideal. Over time, this distribution can lead to a dispersion of the distribution of magnetic spin vectors. This results in dephasing. For molecules that are not moving, the deviation from ideal relaxation is consistent over time, and the signal can be recovered by performing a spin echo experiment. T2*-weighted sequences are used to detect deoxygenated hemoglobin, methemoglobin, or hemosiderin in lesions and tissues. Diseases with such patterns include intracranial hemorrhage, arteriovenous malformation, cavernoma, hemorrhage in a tumor, punctate hemorrhages in diffuse axonal injury, superficial siderosis, thrombosed aneurysm, phleboliths in vascular lesions, and some forms of calcification.

The magnetic resonance sequence may relate to the succession of pulse sequences and pulsed field gradients a specimen is subjected. By varying the parameters of the pulse sequence, different contrasts may be generated between tissues based on the relaxation properties. In other words, different image weightings may be generated. Moreover, different weightings are possible for different sequences. For instance, BLADE (see below) may be combined with a T1, T2, or STIR weighting.

The inventors have recognized that the step of receiving information from image and non-image data provides valuable insights into the image acquisition parameters, especially for medical images acquired with a magnetic resonance acquisition procedure. This is because the information from non-image data may include the raw data from the measurement in a premeditated scheme, i.e. the pulse sequence parameters, the scanner settings, the patient information, the clinical context, and other relevant metadata. By accessing and analyzing both the image and non-image data, the proposed method may reveal or put the focus on information which more directly relates to the image acquisition process. This may lead to better predictions of the image acquisition information and allow for a more readily automation of the reading and reporting workflow. Specifically, the magnetic resonance sequence and/or the image weighting may provide important cues how the medical image needs to be processed for imaging and what kind of CAD-tools are to be applied on the medical image. This is because a T2* weighted image or a corresponding sequence may indicate a very different underlying clinical question and suggest very different subsequent processing steps as a T1 weighted image.

Of note, the information from non-image data is also advantageous with other image acquisition techniques such as computed tomography, as the method also in those cases may open up an additional layer of information.

According to some examples, the image weighting at least comprises or distinguishes between a T1, T2, T2*, proton density (PD), steady-state free precession (SSFP), Susceptibility-weighted (SWI), Short tau inversion recovery (STIR), Inversion recovery (IR), Double inversion recovery (DIR), Diffusion (DWI), Perfusion (PWI), susceptibility-weighted imaging (SWI), Blood-oxygen-level dependent (BOLD), and/or Time-of-flight (ToF) weighting.

According to some examples, the imaging sequence at least comprises or distinguishes between a spin-echo-sequence, a gradient-echo sequence, an inversion recovery, a MR angiography sequence, a saturation recovery sequence, an echo-planar sequence, a spiral pulse sequence, a in and out of phase imaging sequence, and sub-sequences and combinations of the aforesaid.

According to some examples, the imaging sequence distinguishes between at least two different spin-echo-sequences, in particular, between BLADE and HASTE. In other words, determining the image acquisition information comprises classifying the medical image according to at least two different spin-echo-sequences.

BLADE is a proprietary sequence of the Siemens Healthineers AG which reduces the sensitivity to movement in magnetic resonance scanning. It is a technique that incorporates a k-space trajectory radial in nature and reduces motion artifacts and helps visualize the smallest lesions.

HASTE is a spin-echo sequence trademarked by Siemens Healthineers AG. It is a single-shot technique. This means that data from all of k-space is obtained after a single 90°-excitation pulse.

Distinguishing between spin-echo-sequences such as BLADE and HASTE is important for the subsequent processing steps and, in particular, for selecting the right hanging protocol. At the same time, the differences between real image data of different spin-echo sequences are a subtle making this process difficult. In this regard, the inventors have recognized that such differences can be more readily distinguished from non-image data.

According to an aspect, the step of obtaining the non-image data from the medical image comprises accessing meta-data of the medical image. The meta-data of the medical image can comprise, in particular, a header, further in particular, a DICOM header. The non-image data of the medical image comprises the accessed meta-data.

Medical images may be formatted according to the DICOM format. DICOM (=Digital Imaging and Communications in Medicine) is an open standard for the communication and management of medical imaging information and related data in healthcare informatics. DICOM may be used for storing and transmitting medical images and associated information enabling the integration of medical imaging. A DICOM data object consists of a number of attributes, including items such as the patient's name, ID, etc., and also special attributes containing the image (pixel) data and metadata extracted from the image data. The metadata may be stored in the DICOM header.

Obtaining non-image data from the medical image, especially metadata from a DICOM header, allows for the extraction of valuable information that may not be documented in the patient's medical record (or other non-image data) or derivable from the image data. The metadata can include details like the imaging modality used, settings, and the body part displayed, which are crucial for determining the appropriate processing steps in the diagnostic workflow. Therefor obtaining non-image data from metadata of the medical image can lead to more secure and efficient automation.

According to an aspect, the step of obtaining the non-image data from the medical image comprises extracting DICOM tags of the medical image. The DICOM tags of the medical image can comprise, in particular, a set of attributes that store information about the imaging acquisition. The non-image data of the medical image comprises the extracted DICOM tags. The metadata may comprise DICOM tags.

DICOM tags are, in paricular, a set of attributes that store information about the medical image and its acquisition. Each DICOM tag may consist of a group number and an element number, which identify the type of information contained in the tag. For example, the tag (0010, 0010) contains the patient's name, while the tag (0008, 1030) contains the study description. DICOM tags can be used to provide useful non-image data for various image processing tasks, such as identifying the imaging modality, the body part examined, the scan parameters, and the date and time of the acquisition. Some DICOM tags may be mandatory, while others are optional or conditional depending on the context. DICOM tags can also be modified or anonymized to protect the privacy of the patients or to comply with certain standards or regulations.

According to an aspect, the step of providing a first trained function configured to determine an image acquisition information based on non-image data of medical images comprises providing a natural language processing algorithm configured generate text embeddings from input text data and infer body parts corresponding to text embeddings by mapping the generated text embeddings to a learned embedding space According to an aspect, the step of providing a first trained function configured to determine an image acquisition information based on non-image data of medical images comprises applying the natural language processing algorithm to meta-data.

The natural language processing algorithm may be a model that uses natural language processing (NLP) techniques to extract and analyze information from non-image data in order to identify an underlying addressed in the non-image data. One of the techniques used by NLP model is to leverage text embeddings to infer the region of the body that is in focus in a medical image. Text embeddings are numerical representations of words or phrases that capture their semantic and syntactic similarities. NLP Ruler can use text embeddings to analyze the DICOM fields such as BodyPartExamined, StudyDescription and Series Description, which provide textual information about the medical image. By comparing the text embeddings of these fields with those of predefined body regions, NLP Ruler can determine the most likely body region that is displayed in the image. For example, the NLP model can infer that "Brain" and "Head" are related terms that indicate the same region of the body, while "Liver" and "Abdomen" are different terms that indicate different regions. This can overcome the errors and lack of specificity of the field BodyPartExamined alone, which may not always reflect the true region of interest or may be missing or inconsistent.

According to some examples, the imaging sequence type is determined from the non-image data using a sequence classifier wherein the sequence classifier leverages reliable DICOM parameters not dependent on human input (unlike Series Description for example). These parameters may include, for example, the repetition time, the echo time, the inversion time, the flip angle, the number of echoes, the k-space coverage, and the scan duration. These parameters can be the input to machine learning algorithms such as random forests that are trained to predict the sequence type in MRI. A random forest is an ensemble method that uses multiple decision trees to classify the input data based on the most frequent outcome of the individual trees. This method can handle high-dimensional and noisy data and can provide estimates of the prediction accuracy and the feature importance.

By using a sequence classifier based on a random forest algorithm, the proposed method may automatically and accurately identify the MR sequence type from the non-image data, and thus facilitate the subsequent image processing steps.

According to an aspect, the step of obtaining the non-image data from the medical image comprises accessing patient data associated with the medical image, in particular, an electronic medical record of the patient, from a database.

The non-image data of the medical image comprises the accessed patient data.

In particular, the step of obtaining the non-image data may comprise searching for non-image information associated with the image data of the medical image in a data base.

Obtaining the non-image information may comprise querying a healthcare information system such as a HIS (hospital information system), a LIS (laboratory information system), an EMR-system (electronic medical record system) and the like for supplementary information of the patient. Such supplementary information may be obtained in the form of one or more EMR-files (electronic medical record-files), for instance. Further, querying healthcare information systems may be based on a patient identifier such as an ID or the patient's name, electronically identifying the patient in the system. The aforementioned database may be comprised in the healthcare information system.

By obtaining non-image data, in particular patient data, associated with the medical image from a database, particularly an electronic medical record, the computer-implemented method for providing an image acquisition information of a medical image can utilize a more comprehensive set of information, in particular non-image data, as input. This holistic approach can significantly improve the accuracy of workflow automation, as it incorporates both visual and contextual patient information. The provided image acquisition information can enable a user to provide a more informed and precise medical assessments in less time.

According to an aspect, the steps of determining the first image acquisition information and determining the second image acquisition information are executed simultaneously.

To put it differently, simultaneous execution of the steps of determining a first image acquisition information and determining a second image acquisition information implies that the image and non-image data are processed in parallel, rather than sequentially. The simultaneous execution to determine the first and second image acquisition information may entail running two trained functions in parallel or in parallel workflows, in particular the first and second trained function, that can process either image or non-image data and determine a first image acquisition information and a second image acquisition information.

Executing the steps of determining the first image acquisition information and determining the second image acquisition information simultaneously allows for a more efficient and time-saving process. The simultaneous execution can lead to quicker integration and analysis of image and non-image data, which is crucial for the automation of medical image processing workflows. It can reduce the time till the image acquisition information is provided and can therefor allow users to work more efficiently in clinical settings where time is of the essence.

According to an aspect, the step of determining the first image acquisition information is executed before the step of determining the second image acquisition information.

In particular, the step of determining the first image acquisition information comprises determining a score, in particular a first confidence score. The score, in particular the first confidence score, indicates a correctness and/or accuracy of the first image acquisition information. The step of determining the second image acquisition information is only performed if the score, in particular the first confidence score fails to exceed a (predefined) minimum, in particular a confidence minimum.

To put it differently, a sequential determination of image acquisition information can be performed by applying a trained function to the non-image data first, and then applying another trained function to the image data only if the non-image data is incomplete, inaccurate or wrong. If the non-image data is sufficient, complete and/or accurate, which may be determined by the user and/or a (first) trained function, the step of determining the image acquisition information is solely based on the first image acquisition information. This means that the image data does not need to be processed further, and the image acquisition information can be determined based on the first image acquisition information. However, if the non-image data is insufficient, incomplete and/or inaccurate, the image data needs to be processed by another trained function, the second trained function.

A sequential determination of image acquisition information can be more efficient especially in scenarios where the analysis of the non-image data already yields a correct and complete result. This way, the image data analysis can be avoided or postponed if the non-image data is sufficient to determine the image acquisition information. Less image data needs to be processed or the computationally costly image data processing can be avoided altogether, which can streamline the method and reduce computational resources. By using the non-image data as the primary source of information, the first trained function can exploit the semantic and contextual information that is often available in the text format. The non-image data can also be easier and faster to process than the image data, which can have high resolution and dimensionality. The image data analysis can then be selectively applied only when the non-image data is not reliable enough, which can save time and resources for the image acquisition information determination process. Additionally, as only the most relevant image data can be processed, leading to potentially faster turnaround times for the entire image processing workflow. Moreover, the sequential approach can also improve the accuracy and robustness of the image acquisition information, as it can use the image data as a secondary source of information that can verify or correct the non-image data. The image data can provide more visual and spatial information that can complement or resolve ambiguities, inaccuracies or errors in the non-image data. Therefore, the sequential approach can balance the trade-off between efficiency and effectiveness in determining the image acquisition information.

According to an aspect, the step of determining the first image acquisition information comprises determining a first confidence score and/or the step of determining the second image acquisition information comprises determining a second confidence score. The first confidence score indicates a correctness and/or accuracy of the first image acquisition information. The second confidence score indicates a correctness and/or accuracy of the second image acquisition information. The step of determining the image acquisition information is only performed if at least one of the first confidence score or second confidence score exceeds a (predefined) confidence minimum.

The first and second confidence score may comprise a numerical value that indicates the correctness and/or accuracy of the determined image acquisition information. To put it differently, the confidence scores reflect a level of certainty for the determination of the image acquisition parameters. Scarce, inaccurate or wrong non-image data, for example, can lead to a low first confidence score. Image data with artifacts can, for example lead to a low second confidence score. Preferably the first and second confidence score may be determined with the same standard or scale. The first and second confidence score, for example, may be a numerical value between 0 and 1 or 0 and 100, with 1 or 100. A value of 100, for example, indicating fully correct and/or accurate image and/or non-image data.

The first and second confidence scores may be derived from the first and second trained function. Alternatively or additionally, to determine the confidence score, another (third) trained function, such as a machine learning model, can be applied to the image data and non-image data obtained from the medical image. The trained function may include feature extraction and classification components that analyze the data and output a confidence score. The confidence scores may then be compared against a predefined numerical value and/or threshold, in particular a confidence minimum. If the confidence score exceeds this threshold, the image acquisition information determined from the non-image data or image data is considered to be reliable enough to proceed with further processing steps.

According to an aspect, the step of determining the image acquisition information further comprises comparing the confidence score, in particular the first confidence score or the second confidence score, to a predefined threshold, in particular the confidence minimum. The step of determining the image acquisition information may comprise determining the image acquisition information based on the first image acquisition information, if the first confidence score is equal to or higher than the threshold, in particular the confidence minimum. The step of determining the image acquisition information may comprise determining the image acquisition information based on the second image acquisition information, if the second confidence score is equal to or higher than the threshold, in particular the confidence minimum.

The first confidence score and/or second confidence score may comprise multiple sub-scores. The first confidence score and/or second confidence score may be determined by calculating a mean value of the sub-scores and/or weighing sub-scores. The sub-scores in particular may refer to an information comprised by the image acquisition information, for example an image acquisition parameter and/or an information about a body part depicted in the medical image. The image acquisition information may comprise a defined data structure comprising data items. According to some examples, first confidence score and/or second confidence score may comprise multiple sub-scores based on the data items. The first confidence score and/or second confidence score, for example, may comprise a first sub-score relating to the imaging modality, a second sub-score relating the body part depicted in the medical image and a third sub-score relating findings in the body part.

According to an aspect, if the first confidence score exceeds a first confidence threshold, in particular the confidence minimum, the step of determining the image acquisition information is solely based on the first image acquisition information.

According to an aspect, if the first confidence score is below a second confidence threshold, in particular the confidence minimum, and the second confidence score is exceeding the second confidence threshold, in particular the confidence minimum, the step of determining the image acquisition information is solely based on the second image acquisition information.

According to an aspect, the step of determining the image acquisition information based on the first image acquisition information and the second image acquisition information is additionally based on the first and second confidence score to determine a scope and/or prioritization of the first image acquisition information and the second image acquisition information for determining the image acquisition information.

The step of determining the image acquisition information may, in particular, be additionally based on the sub-scores of the first and/or second confidence score. If a sub-score of a first confidence score, for example, is higher than a comparable sub-score of a second confidence score, the image acquisition information is solely based on the first image acquisition information or the corresponding data item of the first image acquisition information.

The determination of the image acquisition information based on the confidence score offers a quantitative measure of the reliability of the first and/or second image acquisition information. The confidence scores can be used for prioritizing the processing of image and non-image data of medical images by focusing on those with higher confidence scores, thus potentially saving time and resources. The confidence scores may allow for a threshold-based decision-making process, where steps are only performed if the confidence score exceeds a certain minimum, ensuring that only the most reliable information is used for further processing. Furthermore, the confidence scores can be composed of multiple sub-scores, providing a detailed assessment of different aspects of the image acquisition information and allowing for a reliable and efficient workflow automation. Confidence scores can reduce the risk of processing errors and improve the overall efficiency of the workflow automation by filtering out unreliable data before it enters the diagnostic process performed by the user.

According to an aspect, the step of determining the image acquisition information comprises determining a conformance score of the first image acquisition information and the second image acquisition information. The conformance score indicates an equivalence or conformance of the first image acquisition information and the second image acquisition information. The image acquisition information is determined based on the conformance score.

In particular, the conformance score can indicate the level of alignment between the first and second image acquisition information. The conformance score may comprise a numerical value that indicates the equivalence and/or alignment of the determined image acquisition information. The conformance score, for example, may be a numerical value between 0 and 1 or 0 and 100, with 1 or 100. A value of 100, for example could be indicating equivalent and/or fully aligned image and/or non-image data.

To determine the conformance score, a trained function, such as a machine learning model, can be applied the to the first image acquisition information and the second image acquisition information and/or the image data and non-image data obtained from the medical image. The trained function may include feature extraction and classification components that analyze the information and output a conformance score. The conformance score may then be compared against a predefined numerical value and/or threshold, in particular a conformance minimum. If the conformance score exceeds this threshold, the first and second image acquisition information determined form the non-image data and image data is considered to be reliable enough to proceed with further processing steps.

For example, if the first image acquisition information determined from non-image data suggests that a CT scan was performed using a specific protocol, and the second image acquisition information determined from image data concurs with that finding, the conformance score would be high, indicating a high level of confidence in the image acquisition information. Conversely, if there is a discrepancy between the two sets of information, the conformance score would be lower, signaling a need for further verification or user input to resolve the inconsistency.

Further, the first image acquisition information determined from non-image data and the second image acquisition information determined from image data may be correct and/or accurate, in particular indicated by a high first and second confidence score. However, the conformance score, for example, may still be low indicating a discrepancy between the non-image data and image data despite of the individually high confidence scores.

The conformance score is a measure of the consistency between the image acquisition information obtained from the non-image data and the image data. This beneficial for ensuring the validity of the image acquisition information that is utilized for the ensuing automated processing of medical images.

According to some examples, if the conformance score is below a threshold, in particular a conformance minimum, an alert or a request for user intervention may be outputted. In other words, a low conformance score (below a certain threshold) can act as a mechanism to trigger alerts or user intervention requests. This ensures that only accurate and verified information is used in the subsequent steps of image processing, thereby enhancing the overall efficiency of the workflow.

According to an aspect, if the conformance score is below a conformance minimum additional information associated with the image data of the medical image is obtained. The additional information is obtained by a plurality of steps.

One step is directed to providing a user information based on the conformance score to a user via a user interface. One step is directed to obtain a user input related to the user information from the user via the user interface. One step is directed to determine the image acquisition information based on the user input.

The user information may comprise a numerical value or a graphical indicator reflecting the conformance score of the image acquisition information. The user information may provide feedback to the user about the reliability and accuracy of the image acquisition information, and whether it is sufficient for further processing steps. The user information may also comprise possible actions for the user to take, such as confirming, correcting, supplementing the image acquisition information, or requesting additional information from the database or another source. The user information may also provide the user with a choice between the first and second image acquisition information, as the right input for the image acquisition information, in case of a discrepancy or uncertainty. The user information may therefore, for example, comprise confidence scores and/or information which one of the image acquisition information is deemed to be more accurate. The user information may be displayed in the same interface as a representation of the medical image, or in a separate interface. The user information may be updated dynamically as the image acquisition information changes or is verified. The user input may be received through various interface means, such as keyboard, mouse, touch screen, voice command, or gesture recognition. The user input may update the conformance score and/or the confidence scores and/or the image acquisition information. In particular, the user input may be directed to confirming, correcting, supplementing the image acquisition information, requesting additional information from the database or another source and/or making a selection between the first and second image acquisition information.

Providing the user information and obtaining the user input may help the user to optimize the workflow and ensure the quality of the medical image analysis. By providing feedback, suggestions, and choices to the user, the user information and/or the user input may facilitate the verification and correction of the image acquisition information and reduce the risk of errors or inconsistencies. The user information and user input may thus improve the usability and functionality of the interface for displaying and analyzing the medical image.

According to an aspect, a computer-implemented method for displaying a representation of a medical image is provided. The method comprises a plurality of steps. One step is directed to receiving the medical image from a database. Another step is directed to determining an image acquisition information of the medical image according to the method of any one of the aspects and examples herein described. Another step is directed to generating a representation of the medical image for displaying in a user interface based on the image acquisition information. Another step is directed to displaying the representation in the user interface.

The representation may be generated by processing the medical image wherein the processing depends on the image acquisition information.

The representation may comprise one or more two-dimensional representation images rendered from the medical image. The representation images may comprise a plurality of image pixels. In particular, the representation images may be two-dimensional renderings of the medical image or of different views of the medical image. Two-dimensional renderings may, in general, rely on known rendering procedures, including ray-casting, ray-tracing, texture-rendering or the like. Thereby, the views and the rendering may depend on the image acquisition information. For instance, the image acquisition information may suggest a certain rendering or pre-processing and/or a particular view.

Further, the representation may comprise a graphical user interface in which the representation images are included at a predefined position. In particular, the graphical user interface may be configured to derive a medical diagnosis based on the medical image. Via the graphical user interface, the user may inspect the medical image, make measurements and record a medical diagnosis (e.g., in the form of a medical report).

By automatically generating the representation based on the image acquisition parameters views are automatically generated and offered to a user which will likely be required based on the image acquisition information. This relives the user from the routine but tedious task of setting up the representation for the further diagnosis of the medical image.

According to an aspect, the step of generating comprises determining a displaying setting based on the image acquisition information and applying the selected displaying setting for generating the representation, the displaying setting being selected from: a contrast setting, a brightness, an intensity windowing, an image enhancement, a look-up table, a viewing plane, a segmentation mask, a zoom level or panning, and/or a volumetric rendering parameter.

By automatically, determining displaying settings, the user is automatically provided with appropriate parameters and does not have to set these herself.

According to an aspect, the representation comprises a volumetric rendering of the medical image, in particular, generated a path-tracing- or ray-casting-based rendering process, and the displaying setting comprises a volumetric rendering parameter for generating the volumetric rendering.

In ray casting, simulated rays emanating from the eye of an imaginary observer are transmitted through the examined body or the examined object (cf. Levoy: "Display of Surfaces from Volume Data", IEEE Computer Graphics and Applications, issue 8, no. 3, May 1988, pages 29-37). Along the rays, RGBA values are determined for sampling points from the voxels and combined to form pixels for a two-dimensional image by means of alpha compositing or alpha blending. Here, the letters R, G and B in the expression RGBA represent the color components red, green and blue, from which the color contribution of the corresponding sampling point is composed. A represents the ALPHA value, which represents a measure for the transparency at the sampling point. The respective transparency is used in the superposition of RGB values at sampling points to form the pixel. Lighting effects are usually taken into account by means of a lighting model within the scope of a method referred to as "shading".

A further method for volume rendering is the so-called path tracing method (cf. Kajiya: "The rendering equation", ACM SIGGRAPH Computer Graphics, issue 20, no. 4, August 1986, pages 143-150). Here, a plurality of simulated rays is shot into the volume data per visualization pixel, said simulated rays then interacting with the volume, i.e., are reflected, refracted or absorbed, wherein at least one random ray is generated every time (except in the case of absorption). Each simulated ray thus finds its path through the volume data. The more virtual rays are used per visualization pixel, the better the image. Here, use can be made, in particular, of the processes and methods described in EP 3 178 068 B1. The content of EP 3 178 068 B1 is incorporated herein in full by reference.

Accordingly, the displaying settings may specify parameters for the path - and/or ray-casting process such as zoom levels, viewing angles, transfer functions, texture values, number of rays, transparency levels, scene illuminations and so forth.

On the one hand, such methods allow particularly realistic visualizations to be generated. This provides the human recipient with an instructive picture of the imaging examination and its outcome. On the other hand, since the volumetric image rendering is triggered automatically, the user does not need to get involved which spares the user of familiarizing herself with the subtleties of a volumetric rendering pipeline (which may be complex).

According to an aspect, the method further comprises selecting, based on the image acquisition information, a hanging protocol including a rule set for displaying one or more representations of a medical image in a user interface, wherein, in the step of displaying, the representation is displayed based on the hanging protocol.

Current reading and reporting systems use general techniques known as "hanging protocols" to format the display or layout of medical images or excerpts from medical images. Hanging protocols allow a user to specifically set displaying environments according to modality, anatomy, and procedure. Hanging protocols present one or more perspectives or views (e.g., in the form of the aforementioned representations) of the medical image to a user, such as a radiologist. Representations may be grouped and located in a graphical user according to characteristics such as the image acquisition information. In addition, hanging protocols may comprise rules or instructions for obtaining additional information such as comparative medical images acquired before or after the medical image or for applying certain image analysis tools.

According to some examples, the hanging protocol may be selected from a plurality of predefined hanging protocols. The predefined hanging protocols may be configured according to different use case and the step of selecting may comprise identifying a use case based on the image acquisition information and selecting the hanging protocol corresponding to the identified use case.

By selecting the appropriate hanging protocol, the user is automatically provided with a user interface specifically adapted for the image data and the diagnostic task. This not only relieves the user but also automates the image processing towards the provision of a medical diagnosis.

According to an aspect, the method further comprises selecting, based on the image acquisition information, an image processing tool configured to provide an image processing result, apply the selected image processing tool so as to generate the image processing result, and displaying the image processing result in the user interface.

According to some examples, the image processing tools may also be applied to any comparative medical images.

The image processing may be selected from a plurality of available image processing tools. The image processing tools may be specific to certain use cases / image acquisition information. For example, the plurality of image processing tools may comprise tools specific for a certain modality, anatomy and/or procedure. Specifically, there might be specific image processing tools for certain magnetic resonance sequences.

Further, the image processing tool may be selected from one or more segmentation tools, one or more detection/classification tools, one or more change detection tools etc. Accordingly, the image detection result may be selected from: a detection result of a medical finding in the medical image, a classification of a medical finding in the medical image, a segmentation of the medical image, and/or a change detected in the medical image.

By automatically identifying and applying image processing tool, results can be automatically generated. The user is provided with cues for arriving at a medical diagnosis without having to search the library of available tools for appropriate ones.

According to an aspect, the method further comprises retrieving, from the database and based on the image acquisition information, a comparative medical image, processing the comparative medical image so as to generate a comparative representation for displaying in the user interface, and displaying the comparative representation in the user interface.

According to some examples, the comparative medical image may have the same or similar image acquisition information as the medical image. According to some examples, the comparative medical image may be processed in the same way as the medical image. For instance, the comparative medical images may be based on the same magnetic resonance sequence and/or image weighting as the medical image. With that, the medical image can be more readily compared to the medical image by the user and/or the same measurements may be automatically made.

The comparative medical image may relate to the same patient as the medical image. The comparative medical image may relate to a different patient as the medical image. In particular, the comparative medical image may have a degree of similarity to the medical image. The comparative medical image may be obtained from a database of comparative medical images.

In particular, the comparative medical image may be obtained from an electronic medical textbook. The comparative medical image may be associated with a verified medical diagnosis.

By offering a comparative medical image to a user, the user can be provided with additional information for deriving a medical diagnosis.

According to some examples, the medical image was acquired of the patient at a first point in time and the comparative medical image was acquired of the patient at a second point in time different than the first point in time.

In other words, a prior or subsequent medical image of the patient may be automatically retrieved which has comparable image acquisition information. In particular, this may mean that the medical image shows a body part of the patient at the first point in time and the comparative medical image shows the body part at a second point in time. With that, the user can more easily determine a development of the health condition of the patient and can come up with a better diagnosis. In this regard, since the comparative medical image is automatically retrieved based on the image acquisition information, the user is spared from the time-consuming task of having to search the database for appropriate priors.

According to some examples, the method further comprises selecting, based on the image acquisition information, a reporting template for producing a medical report corresponding to the medica image, and providing the reporting template via the user interface.

A reporting template may be a pre-configured data structure or building block or module on the basis of which a structured medical report may be generated. A medical report may be generated based on at least one reporting template.

Selecting the reporting template may comprise a selection from a plurality of reporting templates. Each reporting may be specific to a certain image acquisition information. For instance, a certain reporting template may be associated to magnetic resonance scan of the brain, while another reporting template is associated to a chest CT scan.

Each reporting template may specify one or more data fields which have to be addressed or filled for completing the medical report. Further, a reporting template may comprise one or more pull-down menus with items a user can select. As such, a reporting template may also be conceived as an input form or mask structuring the information to be provided for a given diagnostic task.

According to some examples, the method may further comprise pre-filling the reporting template based on the image acquisition information and/or any other image processing results, e.g., as obtained by applying image processing tools.

By fetching appropriate reporting templates, the user is automatically provided with appropriate template data structures. In turn, the user is relieved from the burden of having to search for correct template data structure on her or his own in potentially vast databases.

According to an aspect, a system for providing an image acquisition information of a medical image is provided. The system comprises an interface unit and a computing unit. The computing unit is configured to receive the medical image via the interface unit, to extract the image data form the medical image, to obtain non-image data associated with the image data of the medical image, to determine the first image acquisition information by applying the provided first trained function to the non-image data, to determine the second image acquisition information by applying the provided second trained function to the image data and to determine the image acquisition information based on the first image acquisition information and second image acquisition information, and to provide the image acquisition information via the interface unit.

According to a further aspect, a system for providing control signals for displaying a representation of a medical image is provided. The system comprises an interface unit and a computing unit. The computing unit is configured to receive the medical image from a database via the interface unit, to extract the image data form the medical image, to obtain non-image data associated with the image data of the medical image, to determine the first image acquisition information by applying the provided first trained function to the non-image data, to determine the second image acquisition information by applying the provided second trained function to the image data and to determine the image acquisition information based on the first image acquisition information and second image acquisition information, to generate control signals for controlling a user interface to display a representation of the medical image, the representation being adapted according to the image acquisition information, and to provide the control signals to the user interface via the interface unit.

The computing unit(s) may be realized as a data processing system or as a part of a data processing system. Such a data processing system can, for example, comprise a cloud-computing system, a computer network, a computer, a tablet computer, a smartphone and/or the like. The computing unit can comprise hardware and/or software. The hardware can comprise, for example, one or more processors, one or more memories and combinations thereof. The one or more memories may store instructions for carrying out the method steps according to the invention. The hardware can be configurable by the software and/or be operable by the software. Generally, all units, sub-units, or modules may at least temporarily be in data exchange with each other, e.g., via a network connection or respective interfaces. Consequently, individual units may be located apart from each other.

The interface unit may comprise an interface for data exchange with a local server or a central web server via internet connection for receiving the medial images.

The user interface adapted to interface with one or more users of the system, e.g., by displaying the result of the processing by the computing unit to the user (e.g., in a graphical user interface) or by allowing the user to adjust parameters for arriving at a medical diagnosis.

According to other aspects, the invention further relates to an integrated data management system comprising the above system and an image archiving system configured to acquire, store and/or forward medical images. Thereby, the interface unit may be configured to receive the medical image data set form the image archiving system. According to some examples, the image archiving system may be realized as a cloud storage or as a local or spread storage, e.g., as a PACS (Picture Archiving and Communication System).

According to other aspects, the systems are adapted to implement the inventive method in their various aspects for providing a candidate medical finding. The advantages described in connection with the method aspects may also be realized by the correspondingly configured systems' components.

According to another aspect, the present invention is directed to a computer program product comprising program elements which induce a computing unit of systems herein described to perform the steps according to one or more of the above method aspects and examples as herein described, when the program elements are loaded into a memory of the computing unit.

According to another aspect, the present invention is directed to a computer-readable medium on which program elements are stored that are readable and executable by a computing unit of systems herein described to perform the steps according to one or more method aspects and examples as herein described, when the program elements are executed by the computing unit.

The realization of the invention by a computer program product and/or a computer-readable medium has the advantage that already existing providing systems can be easily adapted by software updates in order to work as proposed by the invention.

The computer program product can be, for example, a computer program or comprise another element next to the computer program as such. This other element can be hardware, e.g., a memory device, on which the computer program is stored, a hardware key for using the computer program and the like, and/or software, e.g., a documentation or a software key for using the computer program. The computer program product may further comprise development material, a runtime system and/or databases or libraries. The computer program product may be distributed among several computer instances.

According to an aspect, the steps of determining the first image acquisition information and determining the second image acquisition information and/or the step of determining the image acquisition information based on the first image acquisition information and the second image acquisition information are implemented by an image-text deep neural network algorithm or trained function, wherein both image data and non-image data can be inputted into the image-text deep neural network algorithm or trained function. The image-text deep neural network algorithm or trained function can handle image data and non-image data concurrently and determine combined image acquisition information.

The trained function can handle both types of data by using a dual-branch architecture, where one branch processes the image data, and the other branch processes the non-image data. The two branches can share some common layers, such as convolutional layers or attention layers, to extract high-level features from both types of data. The two branches can also have some separate layers, such as fully connected layers or recurrent layers, to capture the specific characteristics of each type of data. The two branches can then be merged by a fusion layer, such as a concatenation layer or a weighted sum layer, to combine the features from both types of data and generate a combined image acquisition information. The fusion layer can also perform some operations, such as normalization or regularization, to ensure the stability and generalization of the trained function. The trained function can be optimized using a loss function, such as a mean squared error or a cross-entropy, which measures the difference between the combined image acquisition information and the ground truth image acquisition information.

According to another aspect, the trained function is configured to perform a multi-modal fusion of the image data and the non-image data, wherein the multi-modal fusion comprises a feature-level fusion, a decision-level fusion, or a combination thereof. The feature-level fusion involves extracting and combining features from both image data and non-image data, such as colors, shapes, textures, words, numbers, symbols, etc. The decision-level fusion involves aggregating and weighting the outputs of separate classifiers or regressors for image data and non-image data, such as probabilities, scores, labels, values, etc. The combination thereof involves applying both feature-level fusion and decision-level fusion in a sequential or parallel manner. The multi-modal fusion enables the trained function to leverage the information and/or compensate missing or inaccurate information of each data type, and to generate a more comprehensive and accurate image acquisition information.

Summarizing aspects of the above, according to an aspect, a computer-implemented method for providing an image acquisition information of a medical image is provided. The method comprises a plurality of steps. One step is directed to obtaining the medical image. Another step is directed to extracting image data from the medical image. Another step is directed to obtaining non-image data associated with the image data of the medical image. Another step is directed to providing an image-text trained function configured to determine an image acquisition information based on non-image data and image data of medical images. Another step is directed to providing the image acquisition information.

A trained function that can handle both types of data allows for a more robust and flexible image acquisition information determination, as it can exploit the complementary information from both sources and account for possible discrepancies or inconsistencies between them. The trained function can also adapt to different scenarios where either image data or non-image data may be missing, incomplete, or unreliable, and provide a reasonable image acquisition information based on the available data. The trained function can be implemented using a machine learning model, such as a neural network, which can learn from both image data and non-image data as inputs and output a combined image acquisition information as a result. The trained function can be trained using a supervised, unsupervised, or semi-supervised learning approach, depending on the availability and quality of the labeled data. The trained function can also be updated or fine-tuned using new data as they become available, to improve its accuracy and reliability over time.

Characteristics, features and advantages of the above-described invention, as well as the manner they are achieved, become clearer and more understandable in the light of the following description of embodiments, which will be described in detail with respect to the figures. This following description does not limit the invention on the contained embodiments. Same components, parts or steps can be labeled with the same reference signs in different figures. In general, the figures are not drawn to scale. In the following:
FIG. 1 schematically depicts a system for providing an image acquisition information and/or a representation of a medical image according to an embodiment,
FIG. 2 schematically depicts a method for providing an image acquisition information of a medical image according to an embodiment,
FIG. 3 schematically depicts data flows in a method for providing an image acquisition information of a medical image according to an embodiment,
FIG. 4 schematically depicts a method for providing an image acquisition information of a medical image according to an embodiment with additional steps,
FIG. 5 schematically depicts a method for providing a representation of a medical image according to an embodiment,
FIG. 6 schematically depicts data flows in a method for providing a representation of a medical image according to an embodiment,
FIG. 7 schematically depicts a graphical user interface for displaying a representation of a medical image ion according to an embodiment,
FIG. 8 schematically depicts a trained function, in particular a first trained function, for determining an image acquisition information of a medical image, and
FIG. 9 schematically depicts a trained function, in particular a second trained function, for determining an image acquisition information of a medical image.

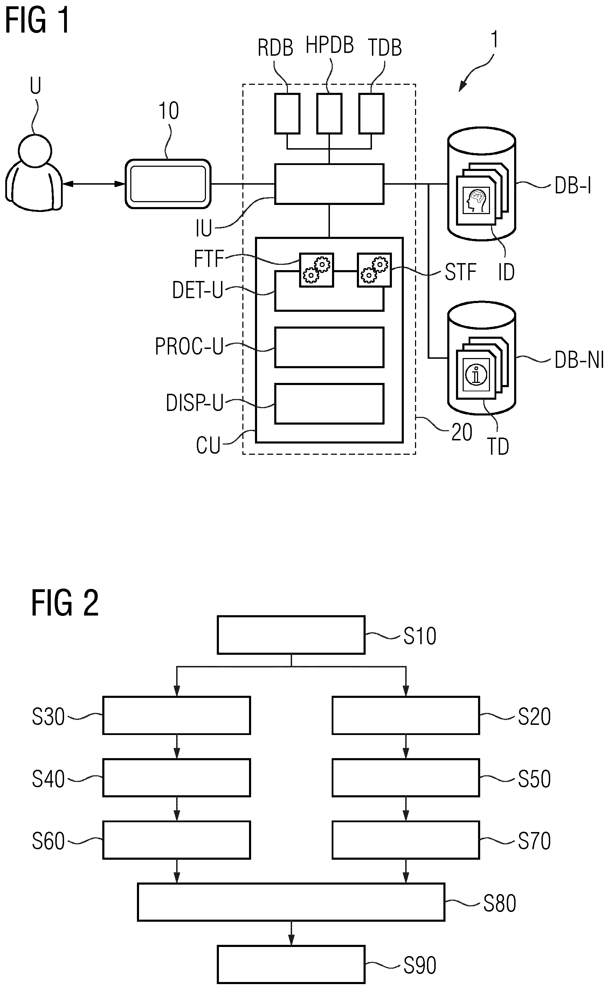

**Figure 1** depicts a healthcare information system 1 for providing an image acquisition information IAI and/or a representation RE of a medical image Ml. In this regard, healthcare information system 1 is adapted to perform the methods according to one or more embodiments, e.g., as further described with reference to Figures 2 to 6.

A user U of healthcare information system 1, according to some examples, may generally relate to a healthcare professional such as a physician, clinician, technician, radiologist and so forth.

Healthcare information system 1 may comprises a user interface 10 and a processing system 20. Further, system 1 may comprise or be connected to one or more databases DB-I, DB-NI generally configured for storing and/or forwarding medical images MI and supplementary (non-image) information. The components of the healthcare information system 1 may also be referred to as "recipients" as they may receive data such as medical images MI and information derived therefrom.

The database DB-I may comprise one or more storage devices for medical images MI which may be realized in the form of one or more cloud storages, local or spread storage modules, e.g., as a PACS (Picture Archiving and Communication System). The database DB-I may generally be configured for storing and/or forwarding the image data ID of the medical images MI. The database DB-II may comprise one or more storage devices for non-image data which may be realized in the form of one or more cloud storages, local or spread storage modules, e.g., as an EMR-system (Electronic Medical Record-system) or RIS (Radiology Information System). The database DB-NI may generally be configured for storing and/or forwarding the non-image data TD of the medical images Ml.

According to some examples, the healthcare information system may comprise one or more medical imaging modalities (not shown) for acquiring medical images Ml, such as a computed tomography system, a magnetic resonance system, an angiography (or C-arm X-ray) system, a positron-emission tomography system, a mammography system, an X-ray system, or the like.

Medical images MI may be three-dimensional image data sets acquired, for instance, using an X-ray system, a computed tomography system or a magnetic resonance imaging system or other systems. The image information may be encoded in a three-dimensional array of m times n times p voxels. Medical images MI may include a plurality of image slices which are stacked in a stacking direction to span the image volume covered by the medical image Ml.

Further, medical images MI may comprise two-dimensional medical image data with the image information being encoded in an array of m times n pixels. According to some examples, these two-dimensional medical images may have been extracted from three-dimensional medical image Ml.

An ensemble of voxels or pixels may be designated as image data of the respective medical image MI in the following. In general, any kind of imaging modalities and scanners may be used for acquiring such image data. Generally, medical images MI may show a body part or an anatomical region or an anatomic object of a patient which may comprise various anatomies and organs. Considering the chest area as a body part, medical images MI might, for instance, depict the lung lobes, the rib cage, the heart, lymph nodes, and so forth.

Medical images MI may be formatted according to the DICOM format. DICOM (=Digital Imaging and Communications in Medicine) is an open standard for the communication and management of medical imaging information and related data in healthcare informatics. DICOM may be used for storing and transmitting medical images and associated information enabling the integration of medical imaging. A DICOM data object consists of a number of attributes, including items such as the patient's name, ID, etc., and also special attributes containing the image pixel data and metadata extracted from the image data. The metadata may be stored in the so-called DICOM header. The metadata of the medical image MI are part of the medical image data and as such may be accessed via database DB-I. However, other non-image data TD associated with the image data ID of the medical image MI is stored in the database DB-NI.

User interface 10 may comprise a display unit and an input unit. User interface 10 may be embodied by a mobile device such as a smartphone or tablet computer. Further, user interface 10 may be embodied as a workstation in the form of a desktop PC or laptop. The input unit may be integrated in the display unit, e.g., in the form of a touch screen. As an alternative or in addition to that, the input unit may comprise a keyboard, a mouse or a digital pen and any combination thereof. The display unit may be configured for displaying representations RE of the medical image MI, medical report templates RT, image processing results Fl, in a graphical user interface GUI, wherein all elements to be shown may be arranged according to a hanging protocol HP.

User interface 10 may further comprise an interface computing unit configured to execute at least one software component for serving the display unit and the input unit in order to provide a graphical user interface for allowing the user to select a target patient's case to be reviewed and making various inputs. In addition, the interface computing unit may be configured to communicate with the database DB or processing system 20 for receiving the medical images MI and any supplementary information. The user U may activate the software component via user interface 10 and may acquire the software component, e.g., by downloading it from an internet application store. According to an example, the software component may also be a client-server computer program in the form of a web application running in a web browser. The interface computing unit may be a general processor, central processing unit, control processor, graphics processing unit, digital signal processor, three-dimensional rendering processor, image processor, application specific integrated circuit, field programmable gate array, digital circuit, analog circuit, combinations thereof, or other now known devices for processing image data. User interface 10 may also be embodied as a client.

The processing system 20 may comprise a computing unit CU and an interface unit IU. Further, the processing system 20 may comprise or be connected to a plurality of dedicated repositories or databases including a reporting database RDB, a tool database TDB, and a hanging protocol database HPDB. According to some examples, the databases RDB, TDB, HPDB may be part of the healthcare information system 1.

The reporting database RDB is a storage device such a cloud or local storage serving as an archive for preconfigured reporting templates RT.

Thereby, a reporting template RT may be seen as a building block for a medical report. Reporting template RT may be configured for editing by the user via user interface 10. Reporting template RT may comprise one or more data fields into which diagnostic information specific for the patient and/or the underlying medical image MI may be specified. The data fields may be empty fields or placeholders for various kinds of data such as text, measurement values or images.

A reporting template RT may be specific to a certain diagnostic use case (which may be indicated by the image acquisition information as herein described).

The hanging protocol database HPDB is a storage device such a cloud or local storage serving as an archive for preconfigured hanging protocols HP.

A hanging protocol HP may comprise a series of rules in the form of computer-executable instructions for optimally arranging medical information, in particular, medical images in a graphical user interface according to a dedicated use case. A hanging protocol may set out which kind of representations of a medical image MI and other information are to be produced and where these elements are to be shown in the graphical user interface GUI.

The tool database TDB is a storage device such as a cloud or a local storage serving as a repository for preconfigured image processing tools IPT.

Image processing tools IPT are generally configured to be applied to medical image data Ml. In other words, these are tools which are configured to process image data in order to provide a corresponding processing result Fl. The image processing result may be related to a medical finding Fl. According to some examples, the processing tools IPT may be specialized for a certain use-case such as a type of medical image data (e.g., MR image data or CT image data) and/or a certain type of image processing result. For instance, one of the image processing tools IPT may be configured to detect lesions in an MR scan of a patient which was acquired with a HASTE sequence, while another image processing tool IPT may be configured to segment bones in a bone window of a CT scan. Generally, the tool database IDB may comprise all image processing algorithms which are available for processing all kinds of image data which may occur at a certain healthcare facility or diagnostic workplace.

Computing unit CU may be a processor. The processor may be a general processor, central processing unit, control processor, graphics processing unit, digital signal processor, three-dimensional rendering processor, image processor, application specific integrated circuit, field programmable gate array, digital circuit, analog circuit, combinations thereof, or other now known device for processing image data. The processor may be single device or multiple devices operating in serial, parallel, or separately. The processor may be a main processor of a computer, such as a laptop or desktop computer, or may be a processor for handling some tasks in a larger system, such as in the medical information system or the server. The processor is configured by instructions, design, hardware, and/or software to perform the steps discussed herein. Further, processing system 20 may comprise a memory such as a RAM for temporally loading the medical images MI and any intermediate processing results. According to some examples, such memory may as well be comprised in user interface 10.

Processing system 20 may comprise sub-units DET-U, PROC-U, DISP-U configured to process the medical image MI and in order to provide an image acquisition information IAI and/or further process the medical image MI based on the image acquisition information IAI.

Sub-unit DET-U is configured to determine image acquisition information IAI for medical images Ml. The image acquisition information IAI comprises the framework under which the medical image MI was acquired. This includes the type of modality and imaging parameters used. Sub-unit DET-U is specifically configured to extract the image acquisition information IAI directly from the image data and non-image data of the medical image Ml. That is, in particular, the pixels and voxels of the medical image MI and text data in the medical image MI and/or text data associated with the medical image Ml. To do so, sub-unit DET-U is configured to extract the image acquisition information IAI from the image data and non-image data of the medical image Ml, such as the pixels, voxels, text data, or metadata. Specifically, sub-unit DET-U is configured to apply one or more trained functions FTF, STF, in particular a first and a second trained function, FTF, STF, to the medical image MI. The trained functions, in particular the first and second trained function FTF, STF, are machine learning models that have been trained to derive the image modality and imaging parameters etc. from different types of data sources, such as image data and non-image data. The first and second trained function FTF, STF comprised by the sub-unit DET-U are configured to determine a first and second image acquisition information FIAI, SIAI. The sub-unit DET-U is further configured to compare and/or verify the first and second image acquisition information FIAI, SIAI to determine the determine image acquisition information IAI.

Sub-unit PROC-U is configured to leverage the image acquisition information IAI for further processing the medical image MI for deriving a medical diagnosis from the medical image MI by a user U. This may involve providing an image processing result by selecting an image processing tool IPT from the tool database TDB according to the image acquisition information IAI and applying it to the medical image Ml. Further, sub-unit PROC-U may be configured to generate one or more representations RE from the medical image MI for displaying to the user U which fit the image acquisition information IAI. Further, sub-unit PROC-U may use the image acquisition information IAI to select suitable reporting templates RT and hanging protocols HP from the reporting database RDB or the hanging protocol database HPDB.

Sub-unit DISP-U is a displaying module or unit. Specifically, sub-unit DISP-U may be configured to use the hanging protocol HP selected by the processing unit PROC-U and arrange any representations RE, image processing results Fl, reporting templates RT, or any other data elements in a graphic user interface GUI according to the hanging protocol HP.

The designation of the distinct sub-units DET-U, PROC-U, DISP-U is to be construed by way of example and not as a limitation. Accordingly, sub-units DET-U, PROC-U, DISP-U may be integrated to form one single unit (e.g., in the form of "the computing unit") or can be embodied by computer code segments configured to execute the corresponding method steps running on a processor or the like of processing system 20. The same holds true with respect to the interface computing unit. Each sub-unit DET-U, PROC-U, DISP-U and the interface computing unit may be individually connected to other sub-units and/or other components of the system 1 where data exchange is needed to perform the method steps.

Processing system 20 and the interface computing unit(s) together may constitute the computing unit of the system 1. Of note, the layout of this computing unit, i.e., the physical distribution of the interface computing unit and sub-units DET-U, PROC-U, DISP-U is, in principle, arbitrary. Specifically, processing system 20 may also be integrated in user interface 10. As already mentioned, processing system 20 may alternatively be embodied as a server system, e.g., a cloud server, or a local server, e.g., located on a hospital or radiology site. According to such implementation, user interface 10 could be designated as a "frontend" or "client" facing the user, while processing system 20 could then be conceived as a "backend" or server. Communication between user interface 10 and processing system 20 may be carried out using the https-protocol, for instance. The computational power of the system may be distributed between the server and the client (i.e., user interface 10). In a "thin client" system, the majority of the computational capabilities exists at the server. In a "thick client" system, more of the computational capabilities, and possibly data, exist on the client.

Individual components of system 1 may be at least temporarily connected to each other for data transfer and/or exchange. User interface 10 communicates with processing system 20 via interface unit IU to exchange, e.g., medical images MI, elements of a graphical user interface GUI or any user input made. Further, processing system 20 may communicate interface unit IU with the database 40 and/or the dedicated database TDB, HPDB, RDB. The interface unit IU may be realized as hardware- or software-interface, e.g., a PCI-bus, USB or fire-wire. Data transfer may be realized using a network connection. The network may be realized as local area network (LAN), e.g., an intranet or a wide area network (WAN). Network connection is preferably wireless, e.g., as wireless LAN (WLAN or Wi-Fi). Further, the network may comprise a combination of different network examples.

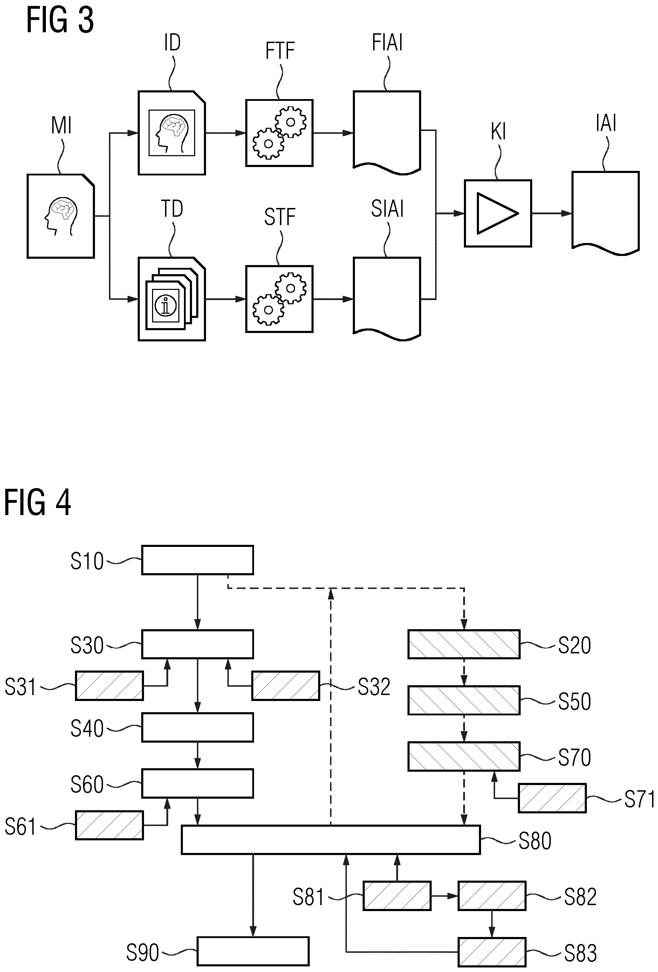

**Figure 2** depicts a method for providing an image acquisition information IAI according to an embodiment. Corresponding data streams are illustrated in **Figure 3****.** The method comprises several steps. The order of the steps does not necessarily correspond to the numbering of the steps but may also vary between different embodiments of the present invention. Further, individual steps or a sequence of steps may be repeated.

**Figure 4** depicts a method for providing an image acquisition information IAI according to an embodiment illustrating several sub-steps. In particular, Figure 4 also illustrates a sequential execution of the steps, in contrast to Figure 2 where various steps, for examples steps S60 and S70, may be executed simultaneously. Furthermore, Figure 4 points out that the steps S20, S50, and S70 are optional, and will be skipped if the non-image data TD already yields sufficient image acquisition information IAI for the medical image Ml.

At step S10, a medical image MI of the patient is obtained. This may involve selecting the medical image MI from a plurality of cases, e.g., stored in the database DB. The selection may be performed manually by the user U, e.g., by selecting appropriate image data in a graphical user interface GUI running in the user interface 10. Alternatively, the medical image MI may be provided to the computing unit CU by the user U by way of uploading the medical image MI to the computing unit CU. According to an example, the medical image MI has been acquired using a magnetic resonance imaging modality using a particular image weighting and magnetic resonance sequence.

At step S20, the image data of the medical image MI is extracted. The image data may be temporarily stored in a digital format at the computing unit CU, e.g., in a working memory of the computing unit CU.

Step S20 may comprise extracting the entire image data of the medical image Ml. According to other examples, step S20 may comprise selecting a representative part of the image data of the medical image MI, in particular, an image slice, and extracting the representative part only.

At step S30, non-image data is obtained for the medical image MI. The non-image data may comprise information about the patient, the imaging device, the scanning parameters, or any other relevant data that is not directly encoded in the image pixels or voxels. The non-image data may be temporarily stored in a digital format at the computing unit CU, e.g., in a working memory of the computing unit CU. The non-image information may contain natural language text. The non-image data may be searched for indications of the image acquisition performed which led to the medical image Ml. This may involve searching the non-image data for (a first) image acquisition information FIAI using the first trained function FTF which may be a natural language processing function, and which may likewise be hosted in the computing unit CU.

At optional sub-step S31, metadata may be extracted from the medical image MI. Metadata may refer to any data that describes or annotates the medical image MI, such as for example its file format, date and time of acquisition, image resolution, modality, contrast agent, etc. Metadata may be stored in a header of the medical image MI, e.g., a DICOM header. The non-image data may be extracted from the header of the medical image MI, in particular a DICOM header. The header may contain metadata such as the patient's name, ID, age, gender, diagnosis history, etc.; the device manufacturer, model, serial number, software version, etc.; the scan date, time, duration, location, etc. and any other annotations or comments made by the operator or the radiologist. Parameters and settings may refer to any data that specifies how the medical image MI was acquired by the imaging device, such as the scan protocol, sequence type, repetition time, echo time, flip angle, field of view, slice thickness, bandwidth, etc. Parameters and settings may be stored in a header of the medical image MI, e.g., a DICOM header. The metadata may be accessed and parsed by the computing unit CU using the first trained function FTF.

At optional sub-step S32, non-image data may be extracted from patient data and/or the medical record of the patient that is associated with the medical image Ml. Patient data and/or the medical record of the patient may refer to any data that provides clinical or demographic information about the patient, such as the patient's name, ID, age, gender, diagnosis history, symptoms, medications, allergies, etc. Patient data may be stored in an electronic medical record (EMR) of the patient, which may be accessed via a network connection, such as an intranet or the internet. The EMR may follow a standard format such as HL7 or FHIR, or a proprietary format of the system provider. The patient data and/or the medical record of the patient may be accessed and parsed by the computing unit CU using appropriate software tools and libraries.

At step S40, a first trained function FTF is provided that is configured to determine an image acquisition information IAI based on non-image data TD. The first trained function FTF may be a machine learning model, such as a neural network, a language model, or any other suitable algorithm, which has been trained on a set of non-image data TD and verified corresponding image acquisition information IAI. The first trained function FTF may be hosted at the computing unit CU or at a remote server that is accessible via a network connection. The first trained function FTF may receive the non-image data TD as an input and output image acquisition information FIAI as an output.

At step S50, a second trained function STF is provided that is configured to determine an image acquisition information IAI based on image data ID of medical images MI. The second trained function STF may be a machine learning model, such as a neural network or any other suitable algorithm, which has been trained on a set of medical images MI or their corresponding image data ID, respectively, and verified corresponding image acquisition information IAI. The second trained function STF may be hosted at the computing unit CU or at a remote server that is accessible via a network connection. The second trained function STF may receive the image data ID of a medical image MI as an input and output image acquisition information SIAl as an output.

At step S60, a first image acquisition information FIAI is determined by applying the first trained function FTF to the non-image data TD. The first trained function FTF may extract relevant features from the non-image data TD that are indicative of the image acquisition information IAI, such as keywords, phrases, numerical values, symbols, or any other data elements. The relevant features may be extracted using natural language processing techniques, such as tokenization, stemming, lemmatization, part-of-speech tagging, named entity recognition, or any other suitable methods. The relevant features may be encoded in a vector or a matrix format that can be input to the first trained function FTF. The first trained function FTF may output a probability distribution over a set of possible image acquisition information IAI categories, such as image modality, image weighting, image sequence, image contrast, image resolution, image orientation, image slice, image region of interest, or any other suitable categories, which may also be designated data items (of the image acquisition information). The first trained function FTF may select the most probable information for each image acquisition information IAI category and/or each relevant feature and/or for the entire non-image data TD. The first image acquisition information FIAI may be configured to output the predefined image acquisition information IAI categories and their corresponding values or labels. The first image acquisition information FIAI may be temporarily stored in a digital format at the computing unit CU.

At sub-step S61, a first confidence score FCS may be determined for the first image acquisition information FIAI that is obtained by applying the first trained function FTF to the non-image data TD of the medical image Ml. The first confidence score FCS may reflect the degree of certainty or reliability of the first image acquisition information FIAI, based on the quality and quantity of the non-image data TD and the performance of the first trained function FTF. The first confidence score FCS may be calculated by the first trained function FTF, or by a separate function, using the probability distribution over the image acquisition information IAI categories and/or the relevant features extracted from the non-image data TD. The first confidence score FCS may be calculated using various methods, such as Bayesian inference, or any other suitable techniques. The first confidence score FCS may be expressed as a numerical value, such as a percentage, a probability, or a score, or as a categorical value, such as high, medium, or low. The first confidence score FCS may be stored in a digital format at the computing unit CU.

At step S70, a second image acquisition information SIAI is determined by applying the second trained function STF to the image data ID of the medical image MI. The second trained function STF may extract relevant features from the image data ID that are indicative of the image acquisition information IAI, such as edges, contours, textures, colors, shapes, regions, or any other visual elements. The relevant features may be extracted using image processing techniques, such as filtering, segmentation, feature detection, feature extraction, or any other suitable methods. The relevant features may be encoded in a vector or a matrix format that can be input to the second trained function STF. The second trained function STF may output a probability distribution over a set of possible image acquisition information IAI categories, such as image modality, image weighting, image sequence, image contrast, image resolution, image orientation, image slice, image region of interest, or any other suitable categories. The second trained function STF may select the most probable information for each image acquisition information IAI category and/or each relevant feature and/or for the entire image data ID. The second image acquisition information SIAl may comprise the selected image acquisition information IAI categories and their corresponding values or labels. The second image acquisition information SIAl may be stored in a digital format at the computing unit CU.

At sub-step S71, a second confidence score SCS may be determined for the second image acquisition information SIAI that is obtained by applying the second trained function STF to the image data ID of the medical image Ml. The second confidence score SCS may reflect the degree of certainty or reliability of the second image acquisition information SIAI, based on the quality and quantity of the image data ID and the performance of the second trained function STF. The second confidence score SCS may be calculated by the second trained function STF, or by a separate function, essentially as described in connection with step S61.

At step S80, an image acquisition information IAI is determined based on the FIAI and, optionally, additionally, on the SIAI. For example, the image acquisition information determined from the image data may be used to verify or correct the image acquisition information determined from the non-image data, or vice versa. For example, a trained function may compare the modality, anatomy, procedure, and/or image acquisition parameters indicated by both the non-image data and the image data and verify if they are matching or divergent. This may comprise inputting the first and second image acquisition information FIAI, SIAI into a trained function TF hosted at the computing unit CU. The trained function TF may be a separate trained function. The trained function TF may output a probability distribution over a set of possible image acquisition information IAI categories and select the most probable information for each category and/or for the entire non-image data and image data. The image acquisition information IAI may comprise the selected image acquisition information IAI categories and their corresponding values or labels.

At sub-step S81, a conformance score may be determined for the image acquisition information IAI that is obtained by combining the first image acquisition information FIAI and the second image acquisition information SIAI. The conformance score may reflect the degree of agreement or consistency between the first image acquisition information FIAI and the second image acquisition information SIAI. The conformance score may be calculated by comparing the probability distributions over the image acquisition information IAI categories output by the first trained function FTF and the second trained function STF, or by comparing the selected values or labels for each image acquisition information IAI category. The conformance score may be calculated using various methods, such as Kullback-Leibler divergence, correlation coefficient, or any other suitable techniques. The conformance score may be expressed as a numerical value, such as a percentage, a probability, or a score, or as a categorical value, such as high, medium, or low. The conformance score may be stored in a digital format at the computing unit CU.

At sub-step S82, an information based on the conformance score may be provided to a user via an interface. The information may indicate whether the first image acquisition information FIAI and the second image acquisition information SIAl are in agreement or not, and to what extent. The information may also suggest or recommend a course of action for the user, such as accepting, rejecting, modifying, or confirming the image acquisition information IAI. The information may be displayed in the interface in a suitable format, such as text, symbols, colors, graphs, or any other visual elements.

At sub-step S83, a user input related to the information may be obtained from the user via the interface. The user input may express the user's decision or preference regarding the image acquisition information IAI, such as whether the user agrees or disagrees with the information, or whether the user wants to change, update, or confirm the information. The user input may also provide feedback or corrections to the image acquisition information IAI, such as adding, deleting, or modifying the image acquisition information IAI categories or values. The user input may be received by the interface in a suitable format, such as text, symbols, colors, gestures, voice commands, or any other inputs. Based on the user input, the image acquisition information IAI may be determined or adjusted accordingly.

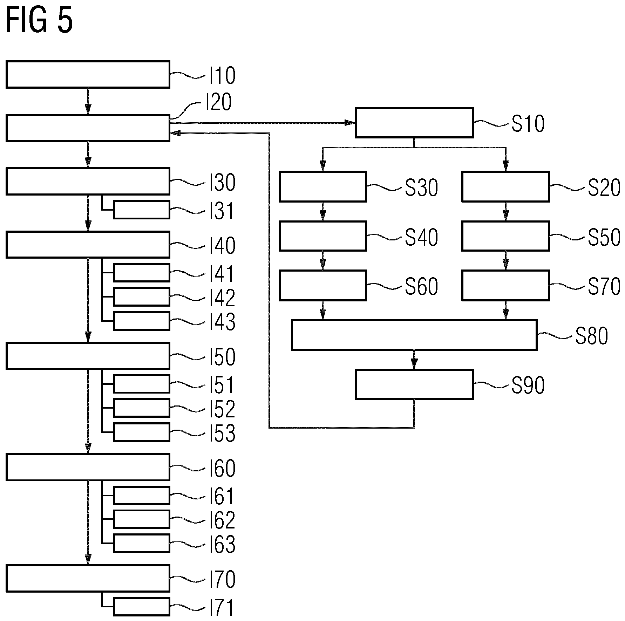

At step S90, the image acquisition information IAI is provided. This may involve showing the image acquisition information IAI in the user interface 10, e.g., in a suitable graphical user interface GUI. Moreover, step S90 may comprise providing the image acquisition information IAI for subsequent image processing steps as shown in connection with Figures 5 to 7.

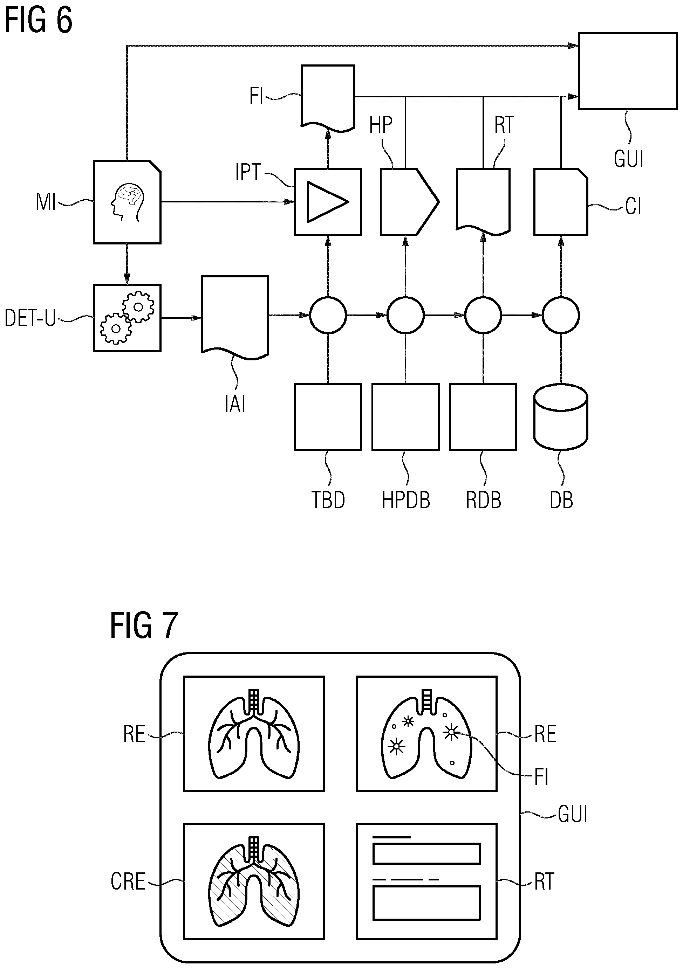

**Figure** 5 depicts a method for displaying a representation RE of a medical image MI according to an embodiment. Corresponding data streams are illustrated in **Figure 6. Figure** 7 shows a corresponding graphical user interface GUI for displaying the representation RE and further information in a reading and reporting workflow. The method comprises several steps. The order of the steps does not necessarily correspond to the numbering of the steps but may also vary between different embodiments of the present invention. Further, individual steps or a sequence of steps may be repeated.

At step 110, the medical image MI is received. Thereby, step 110 substantially corresponds to step S10.

At step I20, the image acquisition information IAI is determined. As shown in Figure 4, this may involve executing steps S20 to S90 as explained in connection with Figures 2 and 3. Optionally, step I20 may further comprise classifying the image acquisition information according to a plurality of predefined diagnostic use cases. According to some examples, the diagnostic uses cases may relate to a diagnostic task a user has to perform or a case group.

At step I30, the image acquisition information IAI is used to generate one or more appropriate representations RE of the medical image MI for displaying to a user U in the user interface 10. This may involve determining the type of representations RE coming into question for the image acquisition information IAI and selecting and processing suited image data from the medical image Ml.

In particular, this may involve determining appropriate display settings for the representation RE (optional sub-step I31). For instance, the display settings may comprise a contrast, brightness, intensity window (e.g., for lung or bone), viewing angle, image enhancement, cinematic rendering parameters and the like.

The display settings may be preconfigured and assigned to certain diagnostic use cases. As the image acquisition information IAI may indicate the diagnostic use case, it becomes possible to determine the display setting based on the image acquisition information IAI.

At step I40, the representation(s) RE generated in step I30 are displayed to the user U in the user interface 10. This may comprise generating appropriate control signals for operating the user interface 10 to display the representation(s) RE.

Optionally, the representation(s) RE may be generated according to a hanging protocol HP. The hanging protocol HP may define what kind of representation(s) RE are to be displayed and where they are to be displayed in a graphical user interface GUI. Further, the hanging protocol HP may set out subsequent processing steps such as the retrieval of comparative images CI or the application of image processing tools IPT. At step I41, a preconfigured hanging protocol HP may be retrieved from the hanging protocol database HPDB which matches the image acquisition information IAI. To this end, a lookup operation may be performed in the hanging protocol database HPDB for a hanging protocol HP corresponding to the image acquisition information IAI. Specifically, an association linking the hanging protocols HP in the hanging protocol database HPDB with image acquisition information IAI or corresponding use-cases may be used to find suitable hanging protocols HP.

At optional step 150, a comparative medical image CI may be retrieved and provided alongside the medical image MI. A comparative medical image CI may generally relate to a medical image which is helpful for arriving at a medical diagnosis based on the medical image MI. As such, the comparative medical image CI may relate to a prior study of the patient. As an alternative, the comparative medical image CI may be a similar image of different patient which may already have been diagnosed. Further, the comparative medical image CI may be an excerpt from an electronic compendium such as an electronic textbook. The type of the comparative medical image CI may be defined in the hanging protocol HP or linked to the diagnostic use case respectively identified based on the image acquisition information IAI.

Specifically, at sub-step I51, the comparative medical image CI may be retrieved from the database DB. Thereby, medical images may be retrieved which match the image acquisition information IAI. For instance, if the image acquisition information IAI indicates that a certain body part was imaged based on a HASTE sequence, the database DB may be searched for medical images which relate to the same body part and at least comparable sequences. To draw such comparison the medical images of a patient in the database coming into question may be subjected to the same processing as the medical image MI for deriving an image acquisition information IAI.

At sub-step I52, the comparative medical image CI may be subjected to an appropriate image processing for preparing a comparative representation CRE therefrom which can be readily compared to the representation RE of the medical image Ml. In particular the same image processing may be applied to the comparative medica image CI which was used for the medical image Ml. In particular, the same display settings may be used.

At sub-step 153, the comparative representation CRE is displayed together with the representation RE. Specifically, the comparative representation CRE may be displayed according to the hanging protocol HP selected at step I41 in the graphical user interface GUI.

At optional step I60, an image processing result FI may be generated based on the medial image MI and provided to the user U via the graphical user interface GUI. The image processing result may be generated according to the image acquisition information IAI and/or the hanging protocol HP. The image processing result FI may relate to a medical finding, a measurement or a segmentation extracted from the medical image MI. The image processing result FI may be generated using a corresponding image processing algorithm or tool IPT which may be executed by the computing unit.

Specifically, at sub-step I61, an image processing tool IPT may be selected from the tool database TDB according to the image acquisition information IAI (or according to the diagnostic use case and/or hanging protocol HP respectively identified based on the image acquisition information IAI). The look-up of the image processing tool IPT may be based on an association linking the image processing tools IPT in the tool database TDB with image acquisition information IAI (or hanging protocols HP / diagnostic use cases).

At sub-step I62, the selected image processing tool IPT may be applied to the medical image Ml. Optionally, at sub-step I62, the image processing tool IPT may also be applied to any comparative medical image CI to obtain a comparative image processing result Fl.

At sub-step I63, the image processing result FI is displayed to the user U in the graphical user interface GUI. The displaying may be in accordance with any rules in the selected hanging protocol specifying the arrangement of image processing results FI in the graphical user interface GUI.

At optional step I70, a reporting template RT on the basis of which medical report may be completed by the user U may be selected and provided. The reporting template RT may be provided to the user U in the graphical user interface GUI. Thereby, the location of the reporting template RT may be determined by the hanging protocol HP.

Specifically, at step I71, a reporting template RT is retrieved from the report database RDB which matches the image acquisition information IAI. To this end, a lookup operation may be performed in the reporting database RDB for reporting template RT corresponding to the image acquisition information IAI. Specifically, an association linking the reporting templates RT with hanging protocols HP or diagnostic use cases (both of which may be identified based on the image acquisition information IAI) may be used to find correct reporting templates RT.

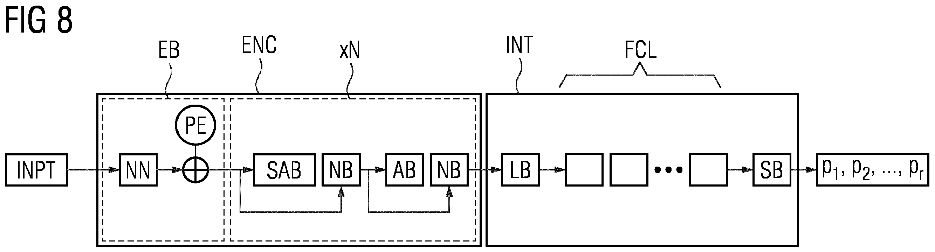

In Figure 8, a schematic representation of the first trained function FTF as a transformer network or model TM is shown. The transformer network TM is configured to process non-image data TD, for example the corpus of text of one or more medical reports of the patient, as input INPT and return indications P₁, P₂,..., Pᵣ of certain data items in the first image acquisition information FIAI. On a high level, the transformer network TM is configured to parse the input text and detect indications for the presence or absence of indications for certain data items which might be relevant for the first image acquisition information FIAI. To fulfill this task, the transformer network has, in particular, learned the ability to deal with synonyms and semantic context such as negations to give only one example.

The transformer network TM according to this embodiment has an encoder ENC and an interpreter INT. In brief, the task of the encoder ENC is to map the input INPT to a sequence of continuous representations R, which is then fed into the interpreter INT. The interpreter INT then maps the representations R to the desired output - in the present case the indications P₁, P₂,..., Pᵣ.

As shown in **Figure 8**, the encoder ENC comprises a plurality of blocks, which may respectively be formed by one or more layers of a neural network.

A first block is configured as embedding block EB. The embedding block EB is configured to transform every word comprised in the input non-image data into a machine-readable format, the so-called input embeddings. Input embeddings, in particular, are real-valued vectors representing the underlying word in the input INPT. In this example, the vectors may have a dimension of 768, that is, each vector of the input embeddings may have 768 entries. Accordingly, the embedding block may have 768 output nodes. The input embeddings encode the meaning of the word such that the words that are closer in the vector space are expected to be similar in meaning. As mentioned, a neural network NN may be used to generate the input embeddings, according to some examples. Specifically, known word-to-vector models may be used as, for instance, described in Mikolov et al., "Efficient Estimation of Word Representations in Vector Space" in arXiv:1301.3781v3, September 7, 2013, the contents of are herein included by reference in their entirety.

Further, in order to capture information about the relative positions of the words in the input INPT, the embedding block EB may be configured to generate positional encodings PE for the input embeddings. The positional encodings PE are of the same dimension as the input embeddings and may be generated using sine and cosine functions of different frequencies. Then, the positional encodings PE may be simply summed to the input embeddings in order to inject the positional information PE in the input embeddings.

The thus generated and modified input-embeddings are input in encoder ENC. The encoder ENC of this embodiment may comprise a stack of N=6 identical blocks. For the sake of easy reference, only one block xN is shown in the drawing. Further, N may also be set to different values and, in particular, to values greater than N=6, according to the respective task. Each block xN of the encoder ENC comprises two subblocks SAB and AB. The first subblock SAB implements a so-called self-attention mechanism. Specifically, the first subblock SAB may be configured to determine how relevant a particular word or input embedding is with regard to other words or input embeddings in the input INPT. This may be represented as an attention vector. In some examples, the first subblock SAB may be configured to compare 1024 words yielding a correspondingly dimensioned attention vector per word. To avoid any bias, multiple attention vectors per word may be generated and fed into a weighted average to compute the final attention vector of every word. There are various ways how the attention mechanism can be implemented in a neural network as, for instance, described in Vaswani et al., "Attention Is All You Need", in arXiv: 1706.03762, June 12, 2017, the contents of which are herein included by reference in their entirety.

The second subblock AB is an adaptation block configured to bring the output of the self-attention subblock SAB into a suitable format for the subsequent processing. The adaptation block AB may comprise a fully connected feed-forward network which may, for example, comprise two linear transformations with Rectified Linear Unit (ReLU) activation in between.

The N=6 layers of the encoder ENC apply the same linear transformations to all the words in the input INPT, but each layer employs different weight and bias parameters to do so. Each subblock SAB, AB is succeeded by a normalization block NB, which normalizes the sum computed between the input fed into the respective subblock SAB, AB and the output generated by the respective subblock SAB, AB itself.

After all layers xN of the encoder ENC have been processed, the results are fed into the interpreter INT. The interpreter INT may comprise a linear block LB which may be another feed-forward layer. It is used to expand the dimensions into a format expected for computing the output vector OUT. That followed, the result is parsed through a plurality of fully connected layers FCL which successively map the processing result of the encoder ENC to the space of output values. Specifically, the last fully connected layer may comprise as many nodes as there are data items in the image acquisition information. In order to provide the indications P₁, P₂,..., Pᵣ. Accordingly, the last fully connected layer generally has r output nodes. Finally, the result of the last fully connected layer may be passed through a softmax block SB for obtaining normalized indications of either 1 or 0.

For training the transformer model TM, a pre-trained encoder ENC is provided. Pre-trained encoders ENC are available for various languages. One advantage of using pre-trained encoders is that they already know the basic semantics of the language the transformer model TM will have to deal with. For an ensuing end-to-end training of the transformer model TM according to the present task of identifying the image acquisition information indications P₁, P₂,..., Pᵣ, a database of 1000 medical images annotated by expert users was used. In particular, the experts determined for each of the 1000 datasets the image acquisition information and/or whether the respective image data and non-image data were sufficient to determine the image acquisition information, indicated by 0 or 1 (ground truth). The database was split into training data (640 datasets), validation data (160 datasets) and test data (200 datasets). For training the transformer model, the backpropagation algorithm was used based on a loss function L(INPT, y₁, y₂,...,yᵣ) = |M(INPT)₁ - y₁|² + |M(INPT)₂ - y₂|² + ... + |M(INPT)ᵣ - yᵣ|², wherein x denotes an input medical image, y₁, y₂,...,yᵣ denote whether on basis of the respective image or non-image data the image acquisition information has been determined (as evaluated by the expert). Furthermore, M(INPT) denotes the result of applying the transformer model TM to the input medical report INPT, and M(INPT)₁, M(INPT)₂, ... , M(INPT)ᵣ correspond to the value of the respective output node of the interpreter INT if applying the machine learning model to the input medical image MI . Based on the validation set of 160 datasets and the corresponding annotations, the best performing transformer model TM out of several transformer models (with different hyperparameters, e.g., number of fully connected layers, stack size N, embedding block EB etc.) was selected. The specificity and the sensitivity were determined based on the test set comprising 200 datasets and the corresponding annotations.

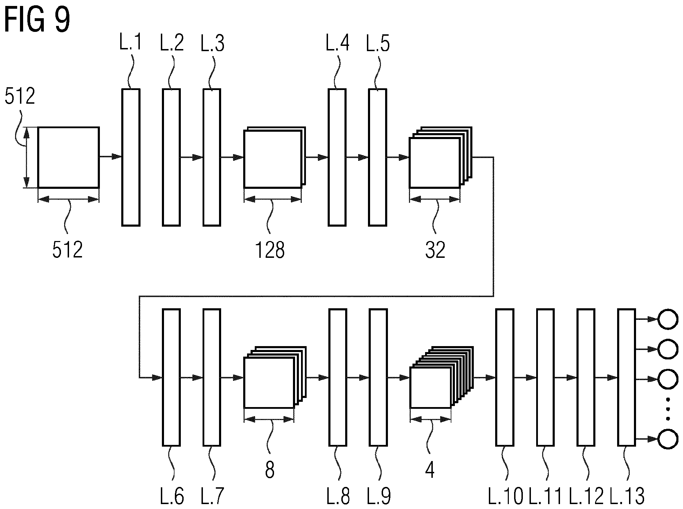

In **Figure 9** an embodiment of the second trained function STF is displayed. In the example shown in Figure 9, the second trained function STF is a convolutional neural network, in particular, a deep convolutional neural network. The second trained function STF according is a machine learning model that can handle different types of input data, especially image data ID. The second trained function STF can, in particular, process pixel or voxel values of the medical image Ml. Of note, this is just meant as an illustrative example as the second trained function STF may also be embodied by any other suitable machine learned model such as transformer architectures or FocalNets as elsewhere herein described.

The second trained function STF according to Figure 9 comprises convolutional layers, pooling layers and fully connected layers. In the input layer L.1, there is one node for data element, e.g. each pixel of the image data, each pixel having one channel (the respective intensity value). After the input layer, there are four convolutional layers L.2, L.4, L.6, L.8, each of the four convolutional layers is followed by a pooling layer L.3, L.5, L.7, L.9. For each of the convolutional layers, a 5x5 kernel is used (indicated by "K: 5x5") with a padding of 2 (indicated by "P: 2") and either one or two filters / convolutional kernels (indicated by "F:1" or "F:2").

From the pooling layers L.3, L.5, L.7, L.9, the first three layers L.3, L.5, L.7 implement an averaging operation over patches of size 4x4, and the last pooling layer L.9 implements a maximum operation over patches of size 2x2. The additional layer L.10 of Figure 7 flattens the input images data. However, this layer is not relevant for the actual calculation.

The last layers of the network are three fully connected layers L.11, L.12, L.13, the first fully connected layer having 128 input and 40 output nodes, the second fully connected layer L.12 having 40 input and 10 output nodes, and the third fully connected layer L.13 having 10 input and 2 output nodes, wherein the 2 output nodes form the output layer of the whole machine learning model.

The value of the first node of the output layer may correspond to one element of the second image acquisition information SIAl (e.g., MR or CT imaging procedure) of the medical image MI related to the input image data ID. The second node may relate to another element of the second image acquisition information SIAI (e.g., spin echo sequence or gradient echo sequence) and so forth. There may be as many output nodes as elements in the second image acquisition information StAt the second trained function STF has to discriminate.

For training the second trained function STF, a database of 500 medical images MI with confirmed image acquisition information IAI has been used. The database was split into training data (320 datasets), validation data (80 datasets) and test data (100 datasets). That followed, the image data ID was extracted from the medical images Ml. For the second image acquisition information SIAI the confirmed image acquisition information IAI are used. For training the second trained function STF, the backpropagation algorithm was used based on a cost function L(x, y₁, y₂,... yₙ) = |M(x)₁ - y₁|² + |M(x)₂ - y₂|² + ... + |M(x)ₙ - yₙ|² wherein x denotes an input image data ID, y₁ denotes whether a first element of the image acquisition information IAI is indicated, y₂ denotes whether a second element of the image acquisition information IAI is indicated, and yₙ denotes whether an n-th element of the image acquisition information IAI is indicated. Furthermore, M(x) denotes the result of applying the second trained function STF to the input image data ID, and M(x)₁, M(x)₂,..., M(x)ₙ correspond to the value of the first, second,... n-th output node if applying the second trained function STF to the input image data.

Based on the validation set of 80 datasets and the corresponding annotations, the best performing second trained function STF out of several machine learning models (with different hyperparameters, e.g., number of layers, size and number of kernels, padding etc.) was selected. The specificity and the sensitivity were determined based on the test set comprising 100 datasets and the image acquisition information IAI.

Wherever meaningful, individual embodiments or their individual aspects and features can be combined or exchanged with one another without limiting or widening the scope of the present invention. Advantages which are described with respect to one embodiment of the present invention are, wherever applicable, also advantageous to other embodiments of the present invention. Independent of the grammatical term usage, individuals with male, female or other gender identities are included within the term.

## Claims

1. Computer-implemented method for providing an image acquisition information (IAI) of a medical image (Ml), the method comprising:
obtaining (S10) the medical image (MI),
extracting (S20) image data (ID) from the medical image (Ml),
obtaining (S30) non-image data (TD) associated with the image data (ID) of the medical image (Ml),
providing (S40) a first trained function (FTF) configured to determine an image acquisition information (IAI) based on non-image data (TD) of medical images (Ml),
providing (S50) a second trained function (STF) configured to determine an image acquisition information (IAI) based on image data (ID) associated with image data (ID) of the medical image (Ml),
determining (S60) a first image acquisition information (FIAI) by applying the first trained function (FTF) to the non-image data (TD),
determining (S70) a second image acquisition information (SIAI) by applying the second trained function (STF) to the image data (ID),
determining (S80) the image acquisition information (IAI) based on the first image acquisition information (FIAI) and the second image acquisition information (SIAl), and
providing (S90) the image acquisition information (IAI).

2. Method according to claim 1, wherein
the image acquisition information (IAI) comprises an image acquisition parameter and/or an information about a body part depicted in the medical image (MI).

3. Method according to any one of the preceding claims, wherein
the step of obtaining the non-image data (TD) comprises accessing meta-data of the medical image (MI), in particular, a header of the medical image (Ml),
wherein the non-image data (TD) comprises the accessed meta-data.

4. Method according to any one of the preceding claims, wherein
the step of obtaining the non-image data (TD) comprises accessing patient data associated with the medical image (MI), in particular, an electronic medical record of the patient, from a database,
wherein the non-image data (TD) comprises the accessed patient data.

5. Method according to any one of the preceding claims, wherein
the steps of determining (S60) the first image acquisition information (FIAI) and determining (S70) the second image acquisition information (SIAI) are executed simultaneously.

6. Method according to any one of claims 1 to 4, wherein
the step of determining (S60) the first image acquisition information (FIAI) is executed before the step of determining (S70) the second image acquisition information (SIAI).

7. Method according to any one of the preceding claims, wherein
the step of determining (S60) the first image acquisition information (FIAI) comprises determining (S61) a first confidence score (FCS) and/or the step of determining the second image acquisition information (SIAI) comprises determining (S71) a second confidence score (SCS),
wherein the first confidence score (FCS) indicates a correctness and/or accuracy of the first image acquisition information (FIAI), wherein the second confidence score (SCS) indicates a correctness and/or accuracy of the second image acquisition information (SIAl), and
wherein the step of determining (S80) the image acquisition information (IAI) is only performed if at least one of the first confidence score (FCS) or second confidence score (SCS) exceeds a confidence minimum (CM).

8. Method according to any one of the preceding claims, wherein
the step of determining (S80) the image acquisition information (IAI) comprises determining a conformance score (QS) of the first image acquisition information (FIAI) and the second image acquisition information (SIAl),
wherein the conformance score (QS) indicates a conformance of the first image acquisition information (FIAI and the second image acquisition information (SIAl), and
wherein the image acquisition information (IAI) is determined based on the conformance score (QS).

9. Method according to claim 8,
wherein, if the conformance score (QS) is below a conformance minimum (QM), additional information associated with the image data of the medical image is obtained, wherein the additional information is obtained by:
providing a user information based on the conformance score to a user via a user interface, and
obtaining a user input related to the user information from the user via the user interface, and
the image acquisition information (IAI) is determined based on the user input.

10. Computer-implemented method for displaying a representation (RE) of a medical image (Ml) comprising,
receiving (I10) the medical image (MI) from a database (DB),
determining (I20) an image acquisition information (IAI) of the medical image (Ml) according to the method of any one of the claims 1 to 9,
generating (I30) a representation (RE) of the medical image (Ml) for displaying in a user interface (GUI) based on the image acquisition information (IAI),
displaying (I40) the representation (RE) in the user interface (GUI).

11. Method according to claim 10, further comprising
selecting (I41), based on the image acquisition information (IAI), a hanging protocol (HP) including a rule set for displaying one or more representations (RE) of a medical image in a user interface (GUI), wherein
in the step of displaying (I40), the representation (RE) is displayed based on the hanging protocol (HP).

12. Method according to any one of claims 10 to 11, further comprising:
selecting (I61), based on the image acquisition information (IAI), an image processing tool (IPT) configured to provide an image processing result (FI),
apply (I62) the selected image processing tool (IPT) so as to generate the image processing result (FI), and
displaying (I63) the image processing result (FI) in the user interface (GUI).

13. System (1) for providing control signals for displaying a representation (RE) of a medical image (Ml) comprising an interface unit (IU) and a computing unit (CU), wherein the computing unit (CU) is configured to:
obtain (S10) the medical image (Ml) via the interface unit (IU),
extract (S20) image data (ID) from the medical image (MI),
obtain (S30) non-image data (TD) associated with the image data (ID) of the medical image (MI),
provide (S40) a first trained function (FTF) configured to determine an image acquisition information (IAI) based on image data (ID) of medical images (Ml),
provide (S50) a second trained function (STF) configured to determine an image acquisition information (IAI) based on non-image data (TD) associated with image data (ID) of the medical image (MI),
determine (S60) a first image acquisition information (FIAI) by applying the first trained function (FTF) to the non-image data (TD),
determine (S70) a second image acquisition information (SIAl) by applying the second trained function (STF) to the image data (ID),
determine (S80) the image acquisition information (IAI) based on the first image acquisition information (FIAI) and the second image acquisition information (SIAI),
generate (I30) control signals for controlling a user interface (GUI) to display a representation (RE) of the medical image (MI), the representation (RE) being adapted based on the image acquisition information (IAI), and
provide (I50) the control signals to the user interface (GUI) via the interface unit (IU).

14. Computer program product comprising program elements which induce a computing unit (CU) to perform steps of the method according to any of claims 1 to 12 when the program elements are loaded into a memory of the computing unit (CU).

15. Computer-readable medium on which program elements are stored that are readable and executable by a computing unit (CU) to perform steps of the method according to any of claims 1 to 12 when the program elements are executed by the computing unit (CU).
